(19) Europäisches Patentamt
European Patent Office
Office européen des brevets

(11) **EP 3 175 782 B1**

(12) **EUROPEAN PATENT SPECIFICATION**

(45) Date of publication and mention
of the grant of the patent:
**05.08.2020 Bulletin 2020/32**

(51) Int Cl.:
***A61B 5/00*** *(2006.01)*     *A61B 5/22* *(2006.01)*
***A61B 5/024*** *(2006.01)*

(21) Application number: **16199822.4**

(22) Date of filing: **21.11.2016**

(54) **METHODS AND APPARATUS FOR DETECTING EXERCISE INTERVALS, ANALYZING ANAEROBIC EXERCISE PERIODS, AND ANALYZING INDIVIDUAL TRAINING EFFECTS**

VERFAHREN UND VORRICHTUNG ZUR ERKENNUNG VON ÜBUNGSINTERVALLEN, ANAEROBE ANALYSE VON ÜBUNGSZEITRÄUMEN UND ANALYSE VON INDIVIDUELLEN TRAININGSEFFEKTEN

PROCÉDÉS ET APPAREIL DE DÉTECTION D'INTERVALLES D'EXERCICE, D'ANALYSE DES PÉRIODES D'EXERCICE ANAÉROBIE ET D'ANALYSE DES EFFETS DE L'ENTRAÎNEMENT INDIVIDUELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2015 US 201562258070 P**

(43) Date of publication of application:
**07.06.2017 Bulletin 2017/23**

(73) Proprietor: **Firstbeat Analytics OY**
**40100 Jyväskylä (FI)**

(72) Inventors:
• **KURUNMÄKI, Veli Pekka**
**40100 Jyväskylä (FI)**
• **MYLLYMÄKI, Tero**
**40100 Jyväskylä (FI)**

• **RUHANEN, Tuukka**
**40100 Jyväskylä (FI)**
• **SAALASTI, Sami**
**40100 Jyväskylä (FI)**
• **SEPPÄNEN, Mikko**
**40100 Jyväskylä (FI)**

(74) Representative: **Kespat Oy**
**Vasarakatu 1**
**40320 Jyväskylä (FI)**

(56) References cited:
**US-A1- 2006 032 315    US-A1- 2014 074 407**
**US-A1- 2014 141 937    US-A1- 2015 250 417**
**US-A1- 2015 327 804**

**Description**

**FIELD**

These methods and apparatus relates to improved recognition of anaerobic sections of exercise and to providing feedback on training load or training effect reflecting energy systems used and trained during exercise.

**BACKGROUND**

Energy for athletic training and exercising of an individual human may originate from anaerobic and aerobic sources. The anaerobic energy is produced when the production of energy from oxygen is not fast enough to meet the demands of external work. The substrates of anaerobic energy systems include energy from Adenosine Triphosphate (ATP) in muscles, Creatine Phosphate (CP) resources, and muscle glycogen. Lactic acid is produced as the end product of anaerobic metabolism but lactic acid can be further oxidized to yield ATP or resynthesized in liver to glycogen. The substrates of aerobic energy systems include muscle glycogen, and circulating glucose and fatty acids. The key determinants of energy production are exercise intensity and time related.

Traditionally, aerobic and anaerobic parts of a specific exercise have been differentiated by heart rate training zones, for example so that heart rate over 90% of individual maximal heart rate has been categorized as anaerobic and under 90% as aerobic training. In more sophisticated solutions, this limit may have been defined by individual anaerobic threshold. However, these solutions lack deep understanding of the relationship between heart rate responses and external work performed and of the physiological responses eliciting training adaptations. Physiologically, when exercise intensity is quickly increased resulting in a need to use anaerobic energy pathways, there can be a mismatch between heart rate and metabolic responses, which need to be considered in order to evaluate anaerobic contributions to energy production and to assess training load accurately.

US-2014/074407-A1, US-2015/250417-A1, US-2014/141937-A1, US-2015/327804-A1, and US-2006/032315-A1 show related methods suitable for analysing anaerobic training.

We present a method and system which relates to recognizing anaerobic parts of exercise from plurality of physiological and physical parameters. These parameters may contain information on physiological responses to exercise and may include heart rate, oxygen consumption, respiration rate, EPOC, TRIMP of which all may be analyzed from heartbeat data. Physical parameters may be external work such as speed, acceleration, power, and also theoretical work (theoretical oxygen consumption) performed. The system can recognize and differentiate anaerobic and aerobic sections from any type of exercise performed without specific exercise protocol (i.e. exercise can be freely performed), provide feedback of characteristics of different exercise sections (intervals), and training load contributing specifically to anaerobic and aerobic performance and energy systems. A method is also presented for estimation of anaerobic training effect. There are no means for estimation of training effect in prior art in supramaximal exercises or exercise where submaximal and supramaximal phases alternate. Only the estimation of training effect in submaximal exercises has been disclosed (applicants own patent US7192401). Since supramaximal training have been found effective in health enhancing purposes and fitness training, it is important to be able to estimate anaerobic training effects too. In addition, in many anaerobic type sports - such as soccer, ice-hockey, alpine skiing etc. - anaerobic training effect may be even more important than aerobic (cardiorespiratory) training effect considering development of physical capacities. Therefore, the invention helps athletes (exercisers) and/or personal trainers and coaches (all users of the invention) to assess more accurately the effects of training, which is crucial for optimizing the content of training from sports-specificity and individuality point of views. In addition, the described invention enables recognition of intervals and estimation of anaerobic training effect in real time. Accordingly, the invention helps in optimizing training dose for athletes or keep fit enthusiasts since coaches and or personal trainers (or exercisers themselves) are able to evaluate whether to continue exercise as planned or whether to increase or decrease intensity. The user can see in real-time the accumulated training load and the impact of training on energy production systems which are intimately related to training adaptations. Considering the analysis of past exercise, it is also useful for the exerciser or coach or trainer to know the number of intervals performed during exercise. In addition, information on the number and intensity of intervals as well as duration of recovery phases together with - or separate from - anaerobic training effect assessment support analysis of physiological training effects.

**SUMMARY**

The invention aims is a method and an apparatus recognize of anaerobic sections of exercise and to provide feedback on training load or training effect reflecting energy systems used and trained during exercise.

The invention is defined by the claims.

The method according to the invention determines an 'oxygen debt' like cumulative physiological sum (usually

EP 3 175 782 B1

training effect TE as EPOC value) brought on by a change in a body homeostasis and its aerobic and anaerobic values. Particularly anaerobic value may be determined by a procedure, where a total EPOC (or TRIMP) is determined as a total sum and an aerobic part calculated in a known manner, is deducted from the total sum. There are two main lines for implementing the invention. The first one scans high intensity phases and recognizes characteristics of each intervals therein using buffering and calculating probabilities to classify intervals. Another implementation uses a different approach, where a starting edge and a starting level of heart rate are main variables to achieve multiplication factor, which converts the measured intensity (%V02max) to the anaerobic intensity. That gives a value for a positive accumulation of anaerobic ' oxygen debt'. A recovery component and scaling may be used to obtain fully repeating results.

Exemplary disclosures of the embodiment may detect exercise intervals, analyze anaerobic exercise periods, analyze training effects and further provide feedback.

In one exemplary embodiment, a heart rate based method and system for recognizing anaerobic sections of exercise and to providing feedback on training load or training effect reflecting energy systems and properties used and trained during an exercise may be conducted according to the following exemplary steps:

a) A user may start to exercise;

b) Heart rate and/or other physiological response of a user may be continuously measured by plurality of physiological parameters, and measured value or values may be recorded with time stamp as physiological data. Physiological parameters may include heart rate. Unreliable data points, such as ectopic beats of heart rate may be filtered or corrected by signal processing first, and remaining points may form accepted data points;

c) Recognition of high intensity intervals based on periods of increasing or decreasing physiological values, for example heart rate. High intensity intervals may be detected by analyzing the derivatives of heart rate with regard to time, i.e. the degree of heart rate changes. This may be done by using a data buffer for storing and analyzing information about the derivatives in real time; The characteristics of each interval are recorded into a buffer. These comprise at least starting %HRmax or %VO2max-value, final peak %HRmax or %VO2max-value and a value depicting the end of the interval as well as timestamps of these parameters The size of the buffer may be 16 records (generally 10 - 30), which means 80s duration when 5s frequency is used in calculation.

d) The probability of the period (interval) to be anaerobic section may be calculated based on the magnitude of the derivatives, heart rate differences, duration of the period, and heart rate level;

e) The found period can thereafter be rejected not to accumulate anaerobic units with specific rules that may be related to

◦ 1) the time difference to the previous anaerobic period being too short if the heart rate level is higher than a certain threshold, for example 70% of HRmax, when the heart rate starts to increase,

◦ 2) the highest heart rate during the period being lower than certain threshold, for example 80% of HRmax,

◦ 3) the duration of the period being shorter than certain threshold, for example 15 seconds,

◦ 4) the highest oxygen consumption value during the period being lower than a certain threshold, for example 73% of VO2max, and

◦ 5) the heart rate difference between the start of the period and the last local heart rate peak value at the end of the period being lower than a predetermined threshold value. The predetermined threshold value (=smallest allowable difference) may be proportional to the HRpeak;

f) Determining the anaerobic sum for the detected periods (high intensity intervals)

g) The anaerobic sum within specified exercise periods (high intensity intervals) that are accepted may be determined based on different factors that can be for example duration of the interval, the peak intensity of the interval as for example %VO2max or %HRmax, the duration of peak intensity of interval, and the physiological recovery status immediately before the interval as %HRmax level of the person. The calculated anaerobic sum may be higher when the interval is shorter, the intensity is higher, and when the person's recovery status before the interval is better, i.e. heart rate is lower;

h) The periods (intervals) can be thereafter also categorized into different groups, for example but not limited to "clear anaerobic", "weak anaerobic", "long" based on quantification of anaerobic sum and duration of the period;

i) Characteristics of different exercise periods can be presented to the user, for example average duration and intensity of intervals.

j) Determining the anaerobic sum performed during steady-state high-intensity periods (=non-interval periods with high intensity) where the anaerobic sum may be cumulative in nature: For example during high exercise intensities, which may be for example 90-100% of HRmax, certain amount of anaerobic sum is cumulatively achieved. It may be related to exercise intensity so that the higher the intensity (i.e. the closer the person is his/her maximal heart rate) the higher anaerobic sum is achieved;

k) Defining the total anaerobic sum for the whole exercise period where the total anaerobic sum by putting together short high intensity intervals and non-interval periods with high intensity;

l) The total amount of anaerobic sum can thereafter be classified by comparing the sum with an anaerobic work scale. For example, a classification may comprise an anaerobic training effect having values between 1-5, and having a verbal description between very easy and very hard (overreaching) anaerobic exercise. Classification may be based on commonly known coaching science, i.e. anaerobic work quantities in different exercises. In addition, physical fitness level of a person may be taken into account when evaluating the anaerobic load of the performed exercise. In principle, a person with higher fitness level (or activity level) needs to get higher anaerobic sum to achieve similar training effect;

m) In similar fashion, the performed aerobic sum is scaled during exercise by comparing measured aerobic sum to reference values for aerobic work. Aerobic sum may be measured with physiological parameters, for example using EPOC and/or TRIMP that are calculated based on heartbeat data. Person's physical fitness level may be taken into account when classifying the calculated aerobic sum. Classification of aerobic sum may comprise aerobic training effect having values typically between 1 and 5, and having a verbal description between minor and overreaching training effect.

n) Further, the proportion of anaerobic effect can be continuously calculated by comparing aerobic and anaerobic training effect -values

Scaling of aerobic and anaerobic training effects

$$1 = \text{Minor training effect / very easy}$$

$$2 = \text{Maintaining training effect / easy}$$

$$3 = \text{Improving training effect / moderate or somewhat hard}$$

$$4 = \text{Highly improving training effect / hard}$$

$$5 = \text{Overreaching training effect / very hard}$$

Following equation (1) may be used to assess the proportion of aerobic and anaerobic training effect (TEaer and TEanaer, respectively) from the overall training effect if both values are under training effect value 5 (Overreaching).

$$proportion\ of\ anaerobic\ effect = \frac{TEanaer}{TEanaer+TEaer}. \qquad (1)$$

If calculated anaerobic sum is higher than required for anaerobic Training effect value 5 (Overreaching), TEanaer can be replaced in the formula by the following equation:

$$TEanaer = \frac{anaerobicSum}{anaerobicSumatTE5level}, \qquad (2)$$

in which anaerobic sum at TE5 level is the sum required to achieve Training Effect value 5 (Overreaching). In a similar fashion, if the aerobic sum defined by EPOC and/or TRIMP is higher than required aerobic sum for Training Effect value 5, TEaer can be replaced in the formula by the following equation:

$$TEaer = \frac{aerobicSum}{aerobicSumatTE5level}. \qquad (3)$$

One exemplary embodiment comprising speed/ altitude or power measurement comprises the following steps:

1. Heart rate and external work output (speed + altitude or power output) are measured during a user performed exercise session

2. Modified intensity (= theoretical VO2) can be calculated using weighted average of heart rate and external workload. External workload can be determined using either the combination of speed and altitude, or power output alone. The resulting value (e.g. ml/kg/min) may be divided by person's maximal oxygen uptake to get proportional intensity (%VO2max) estimate.

    a. It is also possible to calculate modified intensity solely based on external workload. However, combining information on external workload with heart rate in formation may significantly stabilize modified intensity value.

3. Proportional intensity (%VO2max) estimate is calculated based on heart beat data.
4. EPOC value is pre-predicted during the exercise using the %VO2max estimate derived from modified intensity
5. EPOC value is pre-predicted during the exercise using the %VO2max estimate derived from heart beat data
6. Calculating continuously two different Training Effect (TE) estimates based on two different EPOC values
7. Selecting the higher Training Effect value to represent the total Training effect of the exercise or presenting both TE values simultaneously to the user
8. If willing to provide aerobic and anaerobic TE contribution to a user, dividing HR based EPOC estimate by the EPOC estimate derived from work output. Alternatively, HR based TE can be divided by Total TE.

Any of the calculated parameters can be given during and/or after exercise to the user, or to any external system.
The method could be implemented in any device comprising a processor, memory and software stored therein and a user interface, for example, a heart rate monitor, fitness device, mobile phone, PDA device, wristop computer, personal computer, and the like.

## BRIEF DESCRIPTION OF THE FIGURES

Advantages of embodiments of the present disclosure will be apparent from the following detailed description of the exemplary embodiments. The following detailed description should be considered in conjunction with the accompanying figures in which:
The Figures may show exemplary embodiments of the system, method, computer product, and apparatus for detecting exercise intervals, analyzing anaerobic exercise periods, and analyzing individual training effects as herein described. Figures are only exemplary and they cannot be regarded as limiting the scope of invention.

Figure 1 represents a sprint interval exercise where external workload based EPOC (= modified intensity based EPOC; modified intensity can be calculated also solely based on heart rate information) accumulates to a significantly higher level than HR based EPOC. Training session has improving anaerobic training effect.

Figure 2 represents a hard steady pace exercise where external workload based EPOC (= modified intensity based EPOC; modified intensity can be calculated also solely based on heart rate information) accumulates only slightly above HR based EPOC. Exercise has no anaerobic training effect.

Figure 3 represents an example of interval detection wherein exercise consists of a plurality of different intervals. In connection with detection intervals can be classified into different classes based on their characteristics (intensity gradient, peak or average intensity and duration).

Figures 4a-4d represent an example of possible effects of different interval characteristics on accumulation of anaerobic sum: Interval duration, interval peak intensity (%VO2max), HR difference between initial level and highest intensity during the interval, and initial HR level (%HRmax).

Figure 5 represents an example of scaling of anaerobic sum into a training effect value wherein activity class has an effect on the scaling. For the person with higher physical fitness, higher anaerobic sum is needed to achieve similar training effect than for a person with poorer physical fitness level or lower activity level.

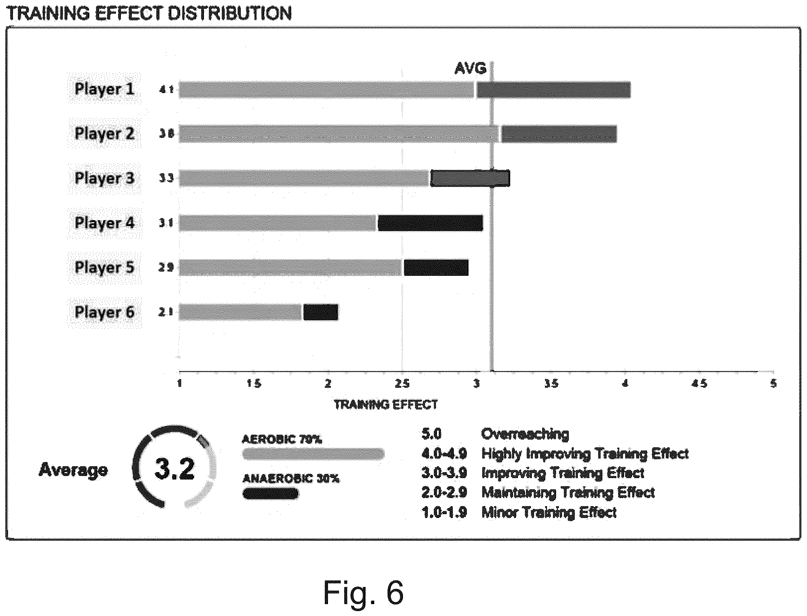

Figure 6 represents an example of user interface with a summary view wherein the contribution of aerobic and anaerobic training effects, and the total training effect of several athletes is shown on a single display.

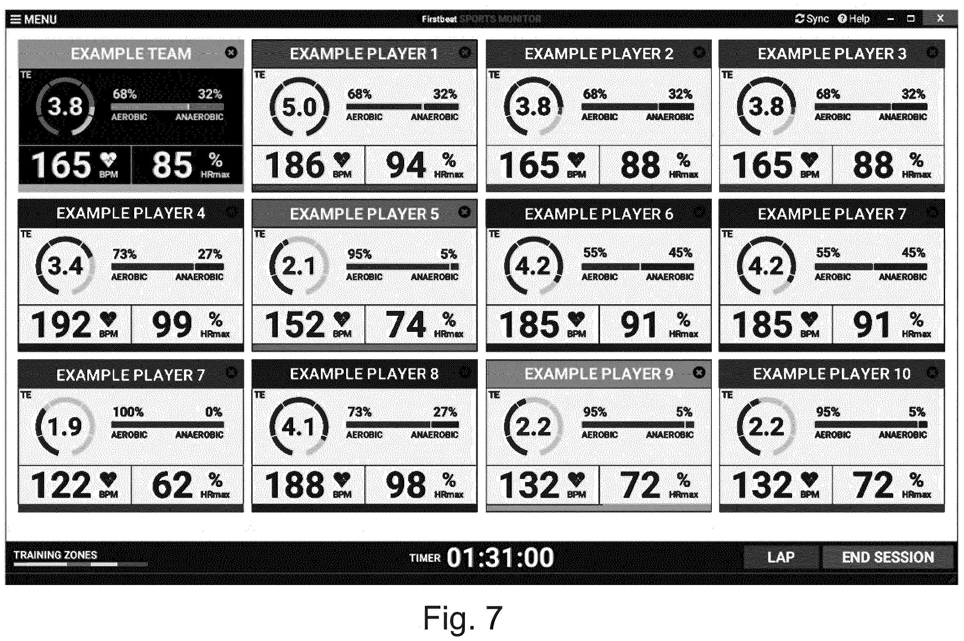

Figure 7 represents an example of user interface with a real time group view wherein the contribution of aerobic and anaerobic training effects, the total training effect, and current exertion level of several athletes is shown on a single display.

Figure 8 represents an example of HR-only based calculation.

Figure 9 represents an example of calculation in situation where both HR and external workload information are available.

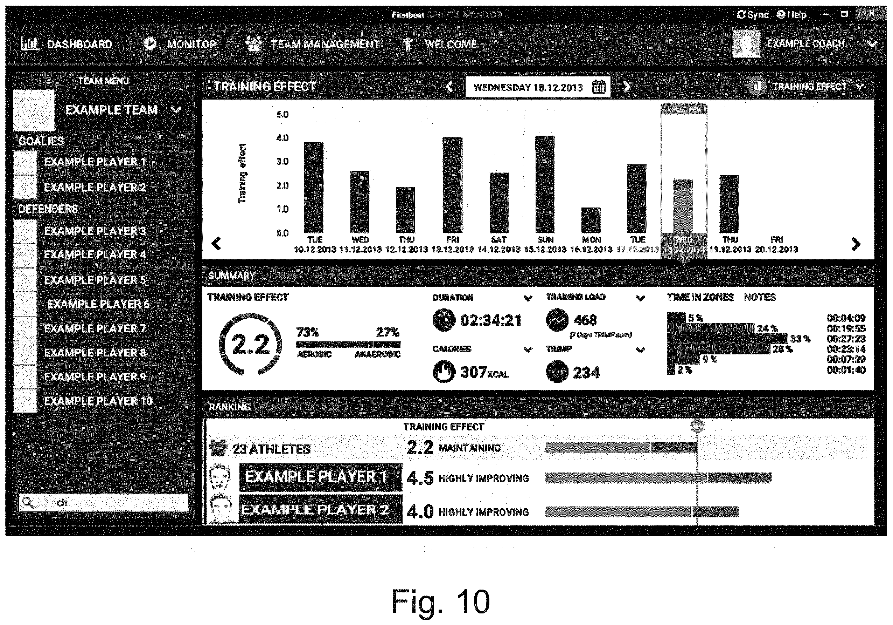

Figure 10 represents an example of user interface with a display that allows comparison of different days as team level averages as well as player rankings within different days.

Figure 11 represents an example of a hardware assembly.

Figure 12 represents an example of embodiment where speed, altitude and HR information are utilized in determining modified intensity. As can be seen, modified intensity can reach much higher values than one what might expect based on HR-level information. As is known from literature MET values correspond to km/h values very well in running. As can be seen from the figure modified intensity matches well with measured speed. Surprisingly, modified intensity corresponds well with speed (km/h) even when it is calculated solely based on heart rate.

Figure 13 represents an example of the accumulation of EPOC as a function of modified intensity.

Figure 14 represents an example of calculation of modified intensity with respect to how it may increase and decrease.

Figure 15 presents Table 1 "Short feedback phrases"

Figure 16 presents Table 2 "Long feedback phrases"

**Table: Exemplary Definitions and Abbreviations**

| Term or abbreviation | Definition |
| --- | --- |
| Anaerobic Training Effect | The physiological impact or effect the training has on anaerobic performance calculated by analyzing the anaerobic sum (e.g. analyzing high-intensity |
|  | intervals) and by scaling that based on commonly known quantities of anaerobic work in different exercises. |
| Anaerobic sum | The physiological stimulus caused by the anaerobic work performed during exercise. Anaerobic sum is calculated by detecting anaerobic sections and continuous high-intensity work from exercise |
| High intensity interval | A period of continuous work during an exercise having typically higher intensity than during rest or recovery periods within exercise. An intermittent (interval-type) exercise typically includes two or more intervals. |
| Clear anaerobic interval | An anaerobic high intensity interval with a higher anaerobic sum than a certain threshold value |
| Weak anaerobic interval | An anaerobic interval with a lower anaerobic sum than a threshold value that is required for a clear anaerobic interval |
| Long interval | An interval that is longer than a certain threshold value, e.g. 200 seconds |
| Aerobic Training Effect | The physiological impact or effect the training has on aerobic performance calculated by analyzing the aerobic sum performed (e.g. using EPOC, TRIMP) and scaling the sum based on commonly known quantities of aerobic work in different exercises |
| Aerobic sum | The physiological stimulus caused by aerobic work performed during exercise and calculated by assessing EPOC and/or TRIMP during exercise |
| Anaerobic threshold = AnT | Anaerobic threshold refers to the highest velocity or external power output that a person's can maintain during physical activity without continuous lactic acid accumulation. AnT can be determined automatically during a user performed high intensity exercise where heart beat interval data and external workload are measured. |
| HR | Heart rate (beats/min) |

(continued)

| Term or abbreviation | Definition |
|---|---|
| HRmax | maximum heart rate (of a person) (beats/min) |
| $\Delta$HR | Change of heart rate level |
| %HRmax | heart rate relative to maximum heart rate |
| VO2 | Oxygen consumption (ml/kg/min) |
| VO2max | maximum oxygen consumption capacity of a person (ml/kg/min) that reflects cardiorespiratory fitness level of the person |
| %VO2max | measured or estimated VO2 relative to VO2max of a person - may be calculated using either HR level information or HRV information |
| RespR | Respiration rate that can be derived e.g. based on heart rate variability measures |
| Theoretical VO2 or theoretical oxygen consumption | Value that describes external workload (ml/kg/min). Can be calculated based on speed and altitude change (or speed and grade of inclination), or based on measured power output in bicycles and other exercise equipment or measured power output during running. |
| on/off -kinetics information | Information related to heart beat data that reflects the rate of change in HR based VO2 estimate |
| $\Delta$t | Refers to instant time or change in time OR duration |
| Activity level / activity class | Refers to person's physical activity level and how much the person is used to exercise. For example, has an effect for following: How much exercise can be tolerated by the person without overstraining one's body OR what is the quantity of exercise that is needed achieve a given training effect. |
| METmax/maxMET/ maximal_MET | maximum oxygen uptake capacity of a person relative to resting oxygen consumption = VO2max (ml/kg/min) / resting VO2 (ml/kg/min) = VO2max (ml/kg/min) / 3,5 ml/kg/min |
| Modified intensity | Intensity estimate that depicts true oxygen requirement during exercise as ml/kg/min or METs or with respect to VO2max (%VO2max). Modified intensity can be calculated using a combination of external workload and heart rate or using only either one of them alone. |
| R-R-interval = RRI | Time interval between successive heart beats in ECG-signal that is measured using e.g. a heart rate monitor. Analysis of R-R intervals (= heart rate variability) allows assessment of e.g. respiration rate in addition to heart rate.<br><br>Measurement of RRI is not mandatory for applying the methods described in this document. Beat-to-beat signal derived from e.g. PPG-signal can be used as well. In addition, all methods can be applied also using heart rate level information from either ECG or PPG signal. |
| HRV | Heart rate variability meaning the variation in time interval between successive heart beats. The magnitude of heart rate variability may be calculated from electrocardiographic or photoplethysmographic signals, for example. |
| Freely performed physical exercise | An exercise that may be performed without a specific protocol. |
|  | The user may freely decide the intensity of exercise, as well as recovery periods inside the exercise session. |
| Continuous measurement | Heart beats may be recorded beat-by-beat or 1-15sec intervals and external power with similar 0.1-15 sec intervals. Calculation of results may be performed with 1-15 sec intervals where 1-5 sec frequency may enable better accuracy. (Selected number of values are recorded) |

(continued)

| Term or abbreviation | Definition |
|---|---|
| Training load | A measure of accumulated load caused by training. The higher the training load the higher is also training stimulus. EPOC and TRIMP are typically used measures of training load. |
| TRIMP (Training impulse) | A cumulative measure describing training load. TRIMP is merely a mathematical index, not a physiological measure |
| EPOC (Excess post-exercise oxygen consumption) | EPOC reflects the extent of disturbance in body's homeostasis brought on by exercise. As it can be nowadays estimated or predicted - based on heart rate or other intensity derivable parameter - it can be used as a cumulative measure of training load in athletic training and physical activity. |
| Non-interval period | Time during exercise that is either recovery phase (or low intensity phase) between high intensity intervals OR high intensity exercising period that cannot be regarded as interval training. |

## DETAILED DESCRIPTION

The invention is defined by the claims. Aspects of the invention are disclosed in the following description and related drawings. Additionally, well-known elements of exemplary embodiments of the invention will not be described in detail or will be omitted so as not to obscure the relevant details of the invention. Further, to facilitate an understanding of the description discussion of several terms used herein follows.

As used herein, the word "exemplary" means "serving as an example, instance or illustration." The embodiments described herein are not limiting, but rather are exemplary only. It should be understood that the described embodiments are not necessarily to be construed as preferred or advantageous over other embodiments. Moreover, the terms "embodiments of the invention", "embodiments" or "invention" do not require that all embodiments of the invention include the discussed feature, advantage or mode of operation.

Further, many of the embodiments described herein are described in terms of sequences of actions to be performed by, for example, elements of a computing device. It should be recognized by those skilled in the art that the various sequences of actions described herein can be performed by specific circuits (e.g. application specific integrated circuits (ASICs)) and/or by program instructions executed by at least one processor. Additionally, the sequence of actions described herein can be embodied entirely within any form of computer-readable storage medium such that execution of the sequence of actions enables at least one processor to perform the functionality described herein. Furthermore, the sequence of actions described herein can be embodied in a combination of hardware and software. Thus, the various aspects of the present invention may be embodied in a number of different forms, all of which have been contemplated to be within the scope of the claimed subject matter. In addition, for each of the embodiments described herein, the corresponding form of any such embodiment may be described herein as, for example, "a computer configured to" perform the described action.

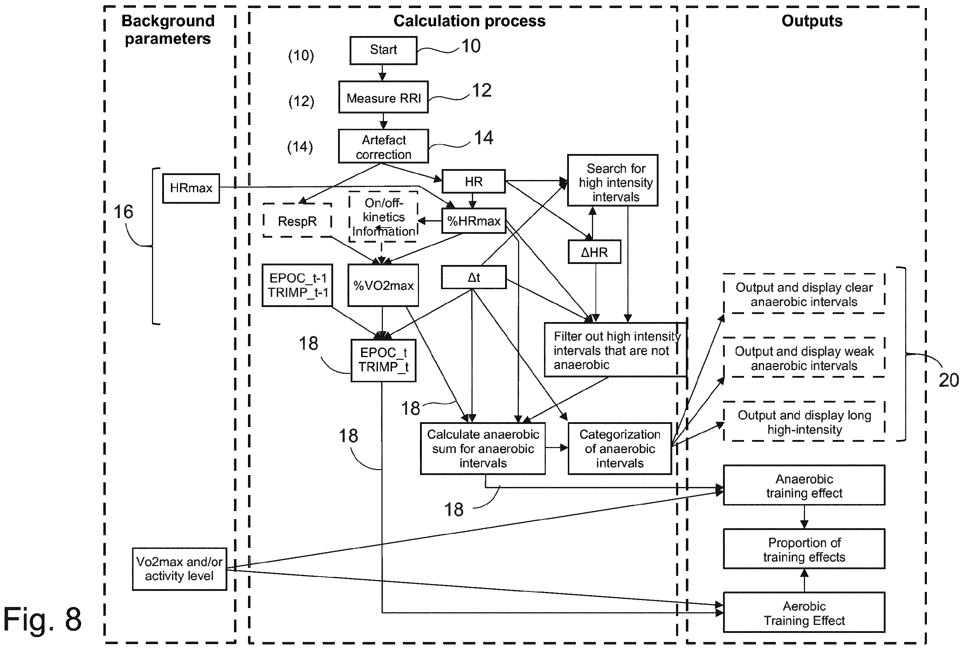

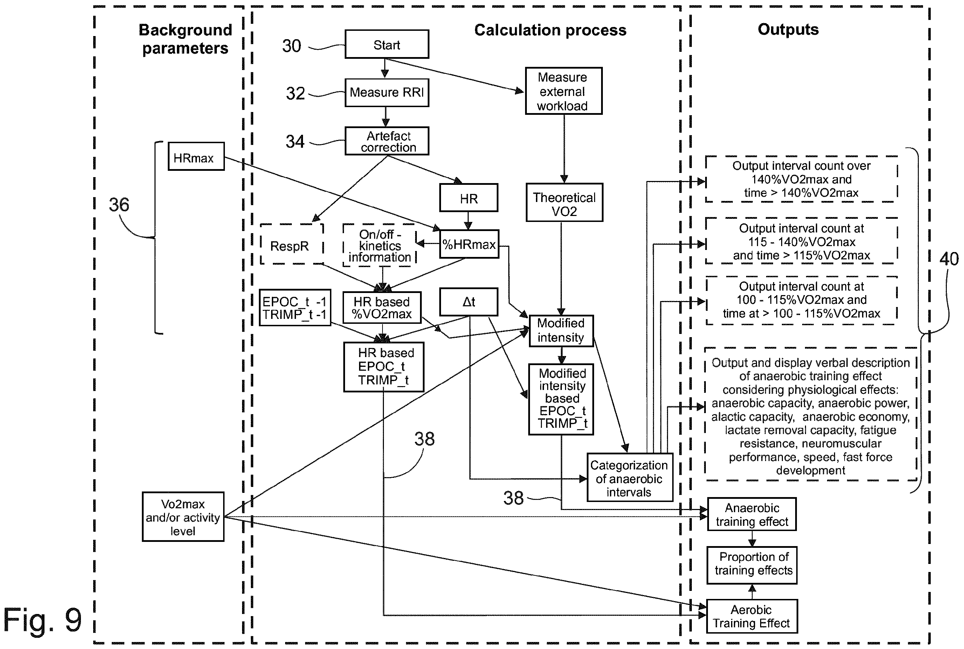

The method can be implemented in versatile devices, which have resources for measuring physiological responses (e.g. Oxygen consumption, heart rate, etc.) and external workload (e.g. speed and altitude or power output), and run software to execute processes depicted in the exemplary flowcharts of Figures 8 and 9. Model considering HR-only based calculation is disclosed in figure 8. The calculation has the following steps:

- User starts measurement (10)
- Beat to beat HR data (for example RR-intervals = RRI) or HR level data is collected (12)
- Artefacts may be detected and corrected (14)
- Searching high intensity intervals from corrected HR signal and filtering out high intensity intervals that are not anaerobic (16)
- Calculating accumulated aerobic sum (e.g. EPOC / TRIMP) as well as anaerobic sum (e.g. EPOC/TRIMP) and determining aerobic training effect and anaerobic training effect based on the sum values (18)
- Interval-count and time in different high intensity training zones can be shown to a user (20)

Calculation comprising both HR and external workload is disclosed in figure 9. The calculation has the following steps:

- User may start a measurement (30)

- Beat to beat HR data (for example RR-intervals = RRI) or HR level data is collected. In addition, external workload is measured for example as speed & altitude or power output time series (32)
- Artefacts may be detected and corrected (34) from HR data
- Calculating modified intensity from corrected HR signal and external workload.
- Calculating accumulated aerobic sum (e.g. EPOC / TRIMP) as well as anaerobic sum (e.g. EPOC/TRIMP) and determining aerobic training effect and anaerobic training effect based on the sum values (38)
- Interval-count and time in different high intensity training zones can be shown to a user (40)

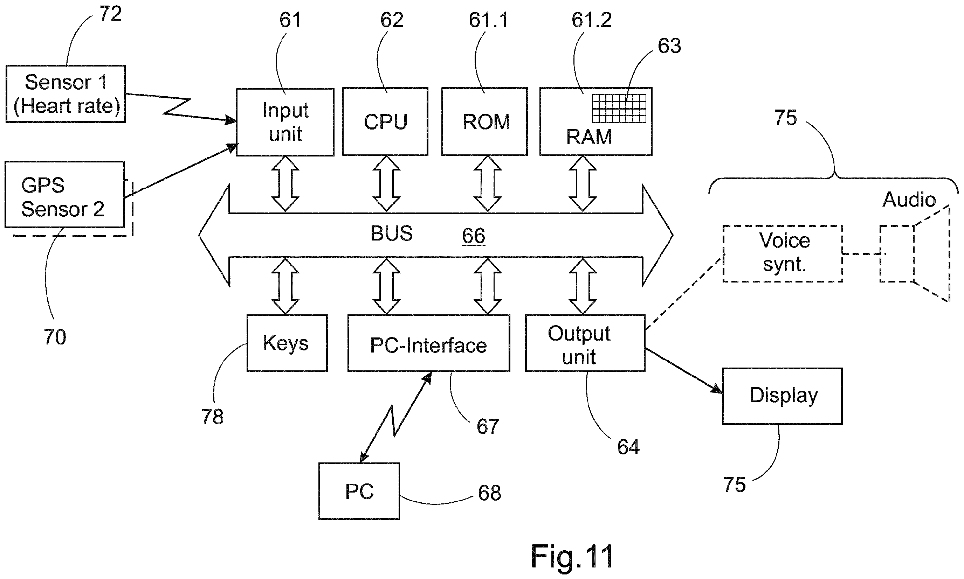

A schematic hardware assembly is depicted in exemplary Figure 11.

The system and method according to the exemplary embodiments can be applied in many kinds of devices as would be understood by a person of ordinary skill in the art. For example, a wrist top device with a heart-rate transmitter, a mobile device such as a phone, tablet or the like, or other system having CPU, memory and software therein may be used.

According to exemplary Figure 11, in the implementation may include an assembly built around a central processing unit (CPU) 62. A bus 66 may transmit data between the central unit 62 and the other units. The input unit 61, ROM memory 61.1, RAM memory 61.2 including a buffer 63, keypad 78, PC connection 67, and output unit 64 may be connected to the bus.

The system may include a data logger which can be connected to cloud service, or other storage as would be understood by a person of ordinary skill in the art. The data logger may measure, for example, physiological response and/or external workload.

A heart rate sensor 72 and any sensor 70 registering external workload may be connected to the input unit 61, which may handle the sensor's data traffic to the bus 66. In some exemplary embodiments, the PC may be connected to a PC connection 67. The output device, for example a display 75 or the like, may be connected to output unit 64. In some embodiments, voice feedback may be created with the aid of, for example, a voice synthesizer and a loudspeaker 75, instead of, or in addition to the feedback on the display. The sensor 70 which may measure external workload may include any number of sensors, which may be used together to define the external work done by the user.

More specifically the apparatus presented in Figure 11 may have the following parts for determining an anaerobic training effect:

- a heart rate sensor 72 configured to measure the heartbeat of the person, the heart rate signal being representative of the heartbeat of the user;
- optionally at least one sensor 70 to measure an external workload during an exercise, and
- a data processing unit 62 operably coupled to the said sensors 72, 70 , a memory 61.1, 61.2 operably coupled to the data processing unit 62,
- the memory may be configured to save background information of a user, for example, background data including an earlier performance level, user characteristics, and the like.

The apparatus may include dedicated software configured to execute the embodiments described in the present disclosure.

In one exemplary embodiment initial background and personal data may be stored. For example, the performance level (for example VO2max or METmax) and the maximum heart rate (HRmax), and the like, of the user may be stored. Personal data may be entered or determined beforehand.

In one exemplary embodiment, a person (e.g. an athlete or keep fit enthusiast) may start an exercise session. The type of exercise can be either interval or continuous, i.e. it can include breaks and rest periods. The user can freely decide the intensity of exercise, as well as recovery periods inside the exercise session. Heartbeat data and performance data can be continuously measured (speed and altitude or power output) during the exercise using, for example, a heart rate monitor, wristop computer or other related device as would be understood by a person of ordinary skill in the art. Even a heartbeat sensor that is connected to a mobile phone or PDA device (using for example Bluetooth connection) can be used, in which case the mobile phone or PDA device would measure external workload (speed and altitude) and serve as a CPU unit.

In further exemplary embodiments the user may exercise outdoors. Both heart rate (or other physiological signal) and external workload can be measured to achieve the most accurate analysis of anaerobic training effects. The user can exercise, for example, by walking, running, or playing sports such as football, rugby, field hockey, tennis, or any other sports. In some embodiments heart rate may be measured using a heart rate transmitter belt, or the like, and analyzed in a CPU-unit that can be, for example, a normal sports watch, wristop computer, or similar device as would be understood by a person of ordinary skill in the art. Alternatively, it may be possible to use ppg (photoplethysmograph)-signal processing so that both the measurement and analysis of data may be done using a wristop device, or the like. Measurement of speed and altitude can be done using a GPS signal. The GPS receiver may be embedded, for example, in the wristop device, but an external GPS receiver can be used as would be understood by a person of ordinary skill in the art. Altitude data can be retrieved from GPS data, additional barometer data, and the like. A barometer may be embedded in the wristop computer. In the described exemplary embodiments a user may, for example, walk or run (or both) during the exercise. The terrain can be whatever the user wants, for example, hilly or flat. During the exercise, data points may be continuously filtered and/or validated. The Training Effect or any parameter calculated by the system can be shown to the user during the exercise, or after exercise, as desired.

In some of the above described exemplary embodiments, heart beat data, speed data and altitude data may be gathered and used, for example, when the user is exercising on foot (walking/pole walking or running) outdoors. In still further exemplary embodiments, a WIFI technique, for example, may be used so that positioning can be performed indoors. It may also be possible to use an accelerometer signal (for example an accelerometer positioned on a user's foot or the like) to define walking/running speed indoors or outdoors, and that data can be used together with barometer data. It is also possible that the exercise is done using a treadmill, or the like. In that case, it is also possible that the speed data can be retrieved from an accelometry signal, or the like. In one exemplary embodiment a user can input treadmill speed data to the CPU while the heart beat data is continuously measured.

. Furthermore, considering the embodiments that use both physiological and external workload data, it is also possible to determine the anaerobic training effect (or other such parameters) in other exercise modes: For example in cycling or rowing power output can be easily measured and retrieved. As would be understood by a person of ordinary skill in the art, power output can be measured in cycling, for example, using a power meter embedded in pedals or chains, and this power data can be shown to the user in a wristop device, or the like. In one exemplary embodiment related to cycling - speed and altitude data may be replaced with power output data measured from a bicycle. The user can do the bicycling exercise indoors or outdoors, and on any desired terrain.

Referring still generally to the exemplary embodiments, where physiological and external workload data are measured, (e.g. cycling or speed and altitude of walking or running are measured) it is possible to increase the accuracy of Training Effect estimate by measuring external workload data. This is because heart beat data can be measured continuously as a function of performance data.

Since purely HR and/or HRV based assessment of anaerobic/aerobic training effect may be beneficial in some cases, these exemplary embodiments are presented below. Purely HR and/or HRV based assessment may be more desired for example in ice-hockey, skating or other sports where external work output is difficult to measure. In addition, positioning indoors is more difficult than outdoors that may lead athletes and coaches to select HR and/or HRV based assessment for indoor exercises.

In one exemplary embodiment disclosing a purely HR and/or HRV based assessment, the system constantly detects exercise intervals from periods of increasing and decreasing heart rate from the heartbeat data. This is done by the system by calculating a moving average of 10 second heart rate difference. The average is calculated for each measurement point by weighting the differences (calculated for the surrounding points) by, for example, a 25 second Hanning window. The averaged heart rate differences are used to define the periods of increasing and decreasing heart rate that follow each other in the data. Of each detected period of increasing or decreasing heart rate, certain parameters are saved in the buffer memory. These are 1) the sum of the averaged heart rate differences during the period of increasing heart rates , 2) the sum of the averaged heart rate differences during the period of decreasing heart rates (the sums are hereinafter denoted by $p$ for heart rate increases and $n$ for decreases), 3) the initial heart rate of the heart rate increase , 4) the initial heart rate of the heart rate decrease (*HRlow* for a heart rate increase and *HRpeak* for a decrease), 5) the time point where *HRlow* or *HRpeak* was measured, as well as 6) the peak intensity as %VO2max at the point of the *HRpeak* value. The aforementioned values are stored in timely order to a constant size data buffer. From the buffer, the oldest values are removed as new periods of increasing and decreasing heart rate are detected or as intervals are detected and/or accepted.

In another exemplary embodiment, the information stored in the data buffer is used to detect exercise intervals when some of the following apply: 1) a maximum amount of increasing and decreasing heart rate periods has been stored in the buffer, or 2) the heart rate level has decreased at least 10 bpm (beats per minute) and the duration of the heart rate decrease has been at least 30 seconds, or 3) the heart rate level drops below 70% of the personal maximum HR. The heart rate data stored in the buffer is used to detect intervals by calculating a value/that represents the interval-likeness of a measured heart rate time sequence. These sequences start from a heart rate increase (buffer index *i*) and end to a heart rate decrease (index *f*). Such sequences are defined for all values of *i* and *f* ($i \leq f$) that are stored in the buffer. The interval-likeness is calculated for each of these sequences. The affecting factors include heart rate derivatives, heart rate differences, and the duration of the sequence. The following formula can be used:

$$I = \left(HR_{peak,f} - HR_{low,i}\right) + p_i + n_f + min\left(p_i, n_f\right) - l/50 - Y, \qquad (4)$$

where $HR_{peak,f}$ is the last local heart rate peak value inside the sequence, and $HR_{low,i}$ is the initial heart rate of the sequence. The duration of the interval $I$ is the duration between $HR_{low,i}$ and $HR_{peak,f}$ in seconds. The sum $p_i$ corresponds to the first heart rate increase in the sequence and $n_f$ corresponds to the last heart rate decrease, and $min(p_i, n_f)$ is the smaller of these two. The term $Y$ describes the effect of the heart rate changes within the sequence and is calculated as

$$Y = \sqrt{\sum_{j=i+1}^{f} p_j^2 + \sum_{k=i}^{f-1} n_k^2}. \qquad (5)$$

In one exemplary embodiment, intervals are accepted for later analysis. For the sequence to be accepted as an interval, certain rules must be fulfilled. These may be 1) the value $I$ must be higher than a threshold value, and it must be greater than those of other time sequences that include some of the same heart rate increases and decreases than the sequence of interest (i.e. the time sequences are partly or totally overlapping), 2) the recovery time from the preceding interval must be longer than 30 seconds or $HR_{low,i}$ must be lower than 70% of the personal maximal heart rate, 3) the highest heart rate of the sequence must be higher than 80% of the personal maximal heart rate, 4) the duration of the sequence must be longer than 15 seconds, 5) the peak value for %VO2max must be higher than 73%, and 6) the difference $HR_{peak,f}$ -$HR_{low,i}$ must be sufficiently high (the required difference is the larger the lower the value of $HR_{low,i}$ is). If the sequence is accepted as an interval, the heart rate information of the sequence and of those preceding it is removed from the data buffer.

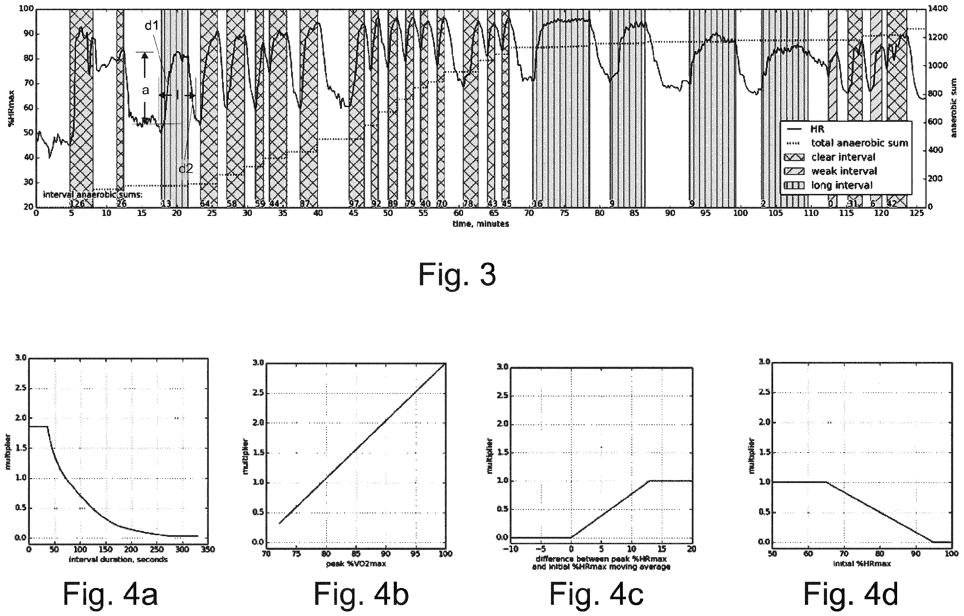

In other exemplary embodiment, detected intervals can be used to calculate accumulated anaerobic sum of exercise. The total anaerobic sum of exercise may be the sum of 1) the anaerobic sum calculated for intervals and 2) the anaerobic sum of long continuous high-intensity exercising. One significant determinant of anaerobic sum may be the duration of the interval. Duration is further multiplied by four factors which describe the properties of the interval (see figure 4). These factors are 1) duration of the interval, 2) peak intensity of the interval as %VO2max, 3) the starting heart rate level of the interval that describes the recovery level, and 4) the difference between the moving HR average at the start of the interval and the HRpeak value inside the interval. The duration affects in principle so that the shorter the duration the higher the multiplier. If the duration of the interval is longer than a maximal threshold value, for example 300 seconds, the effective duration value used in the anaerobic sum calculation can be fixed to the threshold value, so that the anaerobic sum of longer intervals will not be zero. The second multiplier is the higher the higher the peak intensity (%VO2max) of the interval (note that the interval is rejected if the peak intensity is not high enough). The third multiplier is the higher the lower the heart rate is when the interval starts, i.e. the better the recovery level at the onset of the interval. The fourth multiplier is directly proportional to the difference between the HRpeak value and a moving average calculated from the HR values before the start of the interval.

In another exemplary embodiment, in addition to the aforementioned multipliers, also the fluctuations in heart rate within the interval describing noticeable changes in working intensity can be taken into account. The fluctuation can affect the calculated anaerobic sum when the intensity is high enough, for example at least 80% of the personal maximum heart rate. More anaerobic sum can be calculated when there are significant and regular fluctuations in heart rate within an interval. This fluctuation based anaerobic sum can be calculated by the formula

$$\sum_i a \cdot min(n_i, p_i), \qquad (6)$$

where $n_i$ and $p_i$ correspond to the decrease in heart rate before the local minimum heart rate (index $i$) and the increase in heart rate after the local minimum heart rate, respectively. The sum is calculated over the local minimums within the interval.

The factor $a$ can be affected by the factors described in the previous exemplary embodiment.

In another exemplary embodiment, anaerobic sum may be calculated cumulatively from the measured HR data at each point of the exercise even in the case of steady state exercise (= non-interval periods). The amount of the cumulative anaerobic sum can be affected for example by the temporal value of HR signal, time derivative of HR signal, local lowest and peak values of HR signal, average of the HR signal, and personal background parameters (for example anaerobic threshold heart rate, VO2max etc.). For example, when the intensity is above 90 % HRmax, the rate of increase of the cumulative anaerobic sum may be directly proportional to the intensity, so that at 100 % HRmax intensity the cumulative anaerobic sum can increase for example 0.06 units/s. This is in line with physiology since there is always anaerobic metabolism, especially above the anaerobic threshold heart rates or intensities.

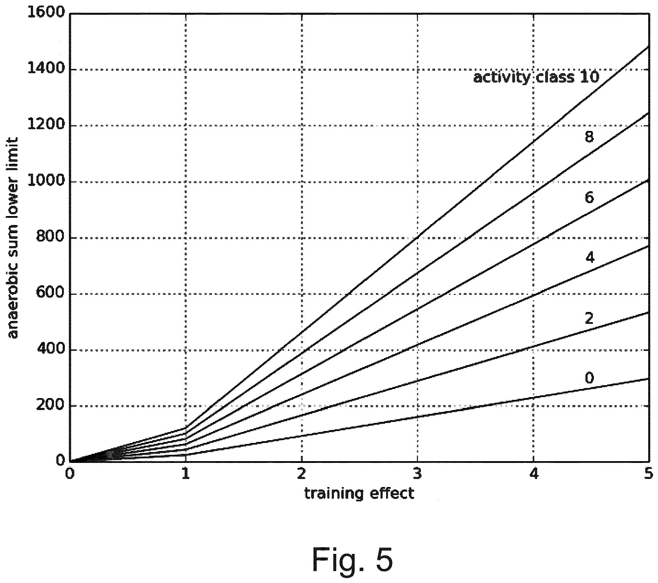

In other exemplary embodiment, anaerobic sum can be used in determining the anaerobic training effect with linear functions. The properties (derivative and zero) of the functions are affected by the user's activity level or fitness level. Examples of such functions can be found in Figure 5. For the person with higher physical fitness, higher anaerobic sum is needed to achieve similar training effect than for a person with poorer physical fitness level or lower activity level.

In one exemplary embodiment, each user's individual anaerobic threshold may be inputted to the system. This may be performed manually, from software or by recognized automatically from exercise parameters (heart rate beat interval data and external workload data required). Individual anaerobic threshold can be used to modify the calculations in order to recognize and take into account more individually the anaerobic work performed. For example, the effect of exercise intensity during intervals can increase the calculated anaerobic sum if the user's anaerobic threshold is lower than default value 90%. In similar fashion, if a person's anaerobic threshold is higher than default 90%, e.g. 93%, less anaerobic sum may be calculated.

In one exemplary embodiment, the time between the detected and/or accepted intervals calculated by the system can be defined to represent recovery time between the intervals.

In one exemplary embodiment, after the intervals have been detected, information regarding the intervals can be provided for the user in real-time or any time after the exercise. These information may include for example number of intervals, intervals distribution to different categories (such as clear anaerobic, weak anaerobic, long interval), the intensity (e.g. average, peak, and lower level of intensity) during intervals, duration of intervals, duration of recovery phases, parameters defining recovery phases (e.g. average, peak, and lower level of intensity), the overall anaerobic sum calculated, the anaerobic sum calculated within intervals, the anaerobic sum calculated outside intervals (i.e. by continuous high-intensity exercising). The information and feedback, of the examples above, can be provided to the user in visual, numerical and verbal form, and this may include all or some of the aforementioned parameters but not limited to these.

In one exemplary embodiment, information on aerobic and anaerobic training effect may be provided to the user. The training effect can include the overall training effect (the highest of aerobic and anaerobic training effect), both or one of the training effects (aerobic and anaerobic), and the distribution of training effect into aerobic and anaerobic.

A practical example of anaerobic sum calculation based on anaerobic interval detection

- Anaerobic interval detection

  ○ During one time period of a high intensity interval training, heart rate (HR) behaves as follows.

    ▪ From 90 bpm to 170 bpm in 1 minute; from 170 bpm to 140 bpm in 30 seconds; from 140 bpm to 165 bpm in 30 seconds; from 165 bpm to 100 bpm in 1 minute.

    ▪ Hereby the duration $I$ of the period is 180 seconds, and the values of the positive and negative changes in HR, $p_i$ and $n_i$, are

    - $p_1 = 80, n_1 = 30, p_2 = 25, n_2 = 65$.

    ▪ The interval likeness of the period can now be calculated according to the equations (4) and (5) as

    $$I = (165 - 90) + 80 + 65 + min(80,65) - 180/50 - \sqrt{25^2 + 30^2} \approx 242$$

  ○ The period is now determined to have the following properties.

    ▪ The interval likeness of the period is higher than an empirically determined threshold value.

    ▪ The interval likeness of the period is higher than any other periods comprising of some of the HR changes inside the period.

    ▪ The recovery time preceding the period (time between the previous potential anaerobic interval and the period) is longer than 30 seconds.

    ▪ The maximum HR value is above 80 %HRmax.

    ▪ The length of the period is between 15 and 200 seconds.

    ▪ The difference between the maximum HR value and the initial HR value is higher than 20 bpm.

  ○ Based on these properties, the period is now validated as a proper anaerobic interval.

- Calculation of the anaerobic sum

○ The anaerobic sum (the "anaerobic effect") of the interval is now calculated by multiplying the duration of the interval, 180 seconds, by the coefficients shown in figures 4a-4d. The affecting coefficients are related to

- Interval duration (coefficient value = 0.25)

- Peak %VO2max (coefficient value = 1.1)

- The difference between peak %HRmax and initial %HRmax moving average, and (coefficient value = 1.0)

- Initial %HRmax (coefficient value = 1.0)

○ After applying the coefficients to the duration of the interval, the resulting anaerobic sum is 49.5.

○ Additional anaerobic sum based on the HR fluctuations is calculated by the equation (6) to be

- $2.5 \cdot min(30,25) = 62.5$

○ Hereby the total anaerobic sum of the anaerobic interval is 49.5 + 62.5 = 112.

The minimum buffered information needed here comprises rise and fall information; %HRmax-differences, timestamps, peak values of %VO2max (or %HRmax) .

In following exemplary embodiments, information on performed external work (e.g. pedaling power in cycling OR speed/ altitude changes in running) can be used to compare theoretical oxygen consumption to heartbeat based oxygen consumption to assess energy provided by anaerobic energy pathways, and to assess training effect achieved using both of the energy pathways. This information can support or substitute the HR/HRV based calculation of anaerobic sum. Of course, use of purely heart rate based estimation of anaerobic and aerobic training effect enables application of the method in all sports. Use of speed and altitude (e.g. running) or power output (cycling, rowing or other exercise equipment) allows even a more detailed analysis of anaerobic training effect. In addition measurement of power during running has recently become possible. Running power can be measured using either speed and altitude OR speed/altitude in combination with acceleration.

One exemplary embodiment comprising speed/ altitude or power measurement comprises the following steps:

1. Heart rate and external work output (speed + altitude or power output) are measured during a user performed exercise session
2. Modified intensity (= theoretical VO2) can be calculated using weighted average of heart rate and external workload. External workload can be determined using either the combination of speed and altitude, or power output alone. The resulting value (e.g. ml/kg/min) may be divided by person's maximal oxygen uptake to get proportional intensity(%VO2max) estimate.

   a. It is also possible to calculate modified intensity solely based on external workload. However, combining information on external workload with heart rate in formation may significantly stabilize modified intensity value.

3. Proportional intensity (%VO2max) estimate is calculated based on heart beat data.
4. EPOC value is pre-predicted during the exercise using the %VO2max estimate derived from modified intensity
5. EPOC value is pre-predicted during the exercise using the %VO2max estimate derived from heart beat data
6. Calculating continuously two different Training Effect (TE) estimates based on two different EPOC values
7. Selecting the higher Training Effect value to represent the total Training effect of the exercise or presenting both TE values simultaneously to the user
8. If willing to provide aerobic and anaerobic TE contribution to a user, dividing HR based EPOC estimate by the EPOC estimate derived from work output. Alternatively, HR based TE can be divided by Total TE.

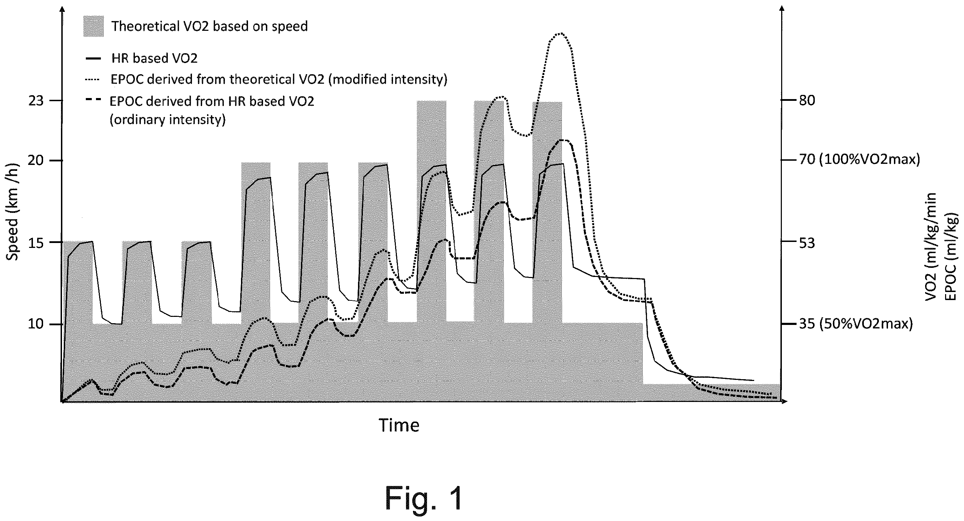

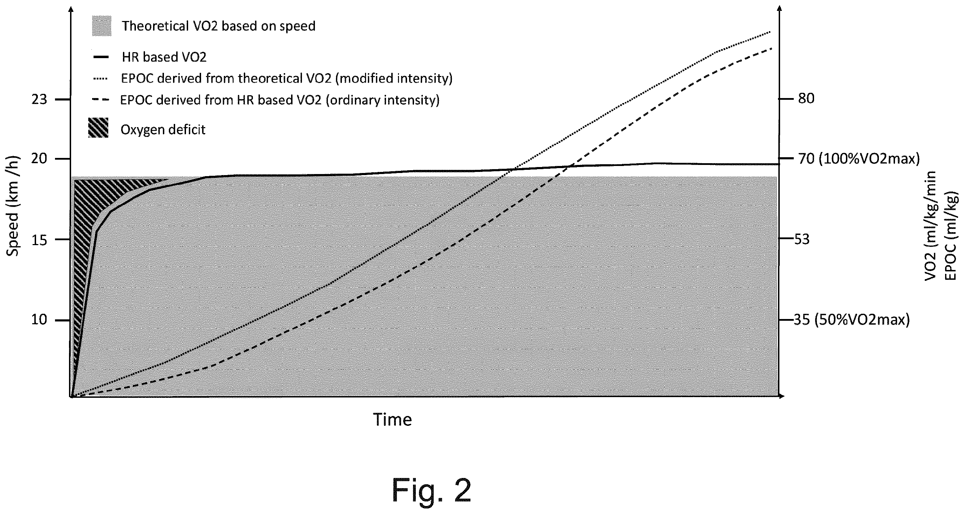

As can be seen from the figure 1, theoretical VO2 based EPOC values get higher values than HR based EPOC values during an interval exercise. The figure presents and interval workout having intervals of 3 x 15km/h + 3 x 20km/h + 3 x 23 km/h wherein each interval is followed by an active recovery period including running with 10km/h speed. Duration of recovery running periods is equal to high intensity running periods. The runner in this example has VO2max of 70ml/kg/min that corresponds to running speed of 20km/h on running track. As can be seen from the figure, when running 20 km/h or slower accumulation of HR based EPOC is only slightly slower when compared with EPOC derived based on Theoretical VO2. This is because HR based VO2 estimate (that reflects the actual VO2 of the runner) reaches theoretical VO2 (that describes the actual need for oxygen for any given speed) during the intervals that are run below VO2max intensity (= 20km/h = 70 ml/kg /min). With higher intensities theoretical VO2 is significantly higher than HR based VO2, which causes the increasing gap between the two EPOC estimates. From a physiological point of view the EPOC value derived based on theoretical VO2 is much more accurate in describing total EPOC since it well reflects the increasing gap between body's oxygen requirement and oxygen supply. The difference in body's oxygen requirement and oxygen supply results in increasing oxygen deficit that is "paid" after exercise as oxygen debt. Actually, EPOC is only partly caused by oxygen debt as there are many other components affecting: for example exercise induced elevated body temperature, respiratory activity, increased level of catecholamine's etc. Calculation method for EPOC is the same but the difference is largely due to the fact that Theoretical VO2 in this example reaches values up to 114% VO2max whereas HR-based VO2 can only reach values up to 100%. The two calculated EPOC peak values are 74 ml/kg and 94 ml/kg for HR based and theoretical VO2 based EPOC, respectively. Accordingly the runner may be shown that total training effect was 3.5 and the effect was 79% aerobic (=74/94) and 21 % (=20/94) anaerobic. An opposite example is presented in figure 2 where same person has performed a hard submaximal steady pace exercise where heart rate based EPOC is 91 ml/kg and theoretical VO2 based EPOC 94 ml/kg. Accordingly, total training effect is 3.5 but aerobic contribution is 97% and anaerobic contribution is only 3%.

Although prior art discloses comparison of used energy systems during exercise it does not provide means to estimate the actual training effect. Actually, comparison of proportions of aerobic and anaerobic energy yield is usually not meaningful since in long exercises most of energy is produced aerobically even if exercise would include hard anaerobic periods. On the contrary, EPOC provides a more sophisticated measure for training effect as it is a well-established measure of training effect. EPOC actually reflects the extent of disturbance in body's homeostasis caused by exercise. EPOC can be modelled, for example, using neural network modelling with a large amount of experimental data.

In such a case, total training effect is calculated using HR based EPOC (that is higher) and the aerobic effect would be 100% and anaerobic effect 0%. This makes sense also from a physiological point of view since actual measured VO2 has a slow component meaning that in prolonged exercises VO2 drifts to a higher level than theoretical VO2.

For example, the following calculation formulas can be used for theoretical VO2:

$$\text{Theoretical VO2 of running (ml/kg/min)} = 0.2 * (\text{speed m/min}) + 0.9 * (\text{speed m/min}) * TAN (\text{grade of incline}) + 3.5$$

$$\text{Theoretical VO2 of walking (ml/kg/min)} = 1.78 * (\text{speed m/s}) * 60 * (TAN (\text{grade of incline}) + 0{,}073)$$

A threshold speed of e.g. 7.5 km/h can be used in switching from walking formula to running formula. Alternatively, detection between walking and running can be used using accelerometer data.

In cycling, power output can be converted to VO2 using the following exemplary formula:

$$\text{Theoretical VO2 of cycling (ml/kg/min)} = ((\text{power watts}) * 12 + 300)) / \text{person's weight}$$

$$\text{Theoretical VO2 (Indoor) rowing VO2 (ml/kg/min)} = (14.72 * Power + 250.39) / \text{person's weight}$$

In addition, equations have been described for the calculation of road cycling power based on measured speed and altitude data etc. based on which %VO2max can be calculated.

In one exemplary embodiment the accuracy of theoretical VO2 calculation is improved in interval type sports. As is known in the art, theoretical VO2 of accelerated or decelerated running at any given speed differ significantly from steady-speed running. For example, during acceleration phase a runner may have an average speed of 15km/h during a sampling period. In this case, for example, if initial speed has been 0 km/h and end speed 30km/h, the average value of 15 km/h provides too low estimate of theoretical VO2. Accordingly, using acceleration as a multiplying factor the error can be avoided.

In one exemplary embodiment HR-only based calculation of anaerobic training effect can also be applied without interval detection. In that case calculation would go as follows:

a) A user starts to exercise;

b) Heart rate (beat-by-beat heart rate or HR-level) of a user is continuously measured and recorded with time stamp,

c) Unreliable data points, such as ectopic beats of heart rate may be filtered or corrected by signal processing first, and remaining points may form accepted data points;

d) Determining user's modified intensity (%VO2max) from data utilizing information on e.g. HR level relative to his/her maximal heart rate (%HRmax), RRI derived respiration (if R-R intervals are available) and heart rate derivative (e.g. %HRmax) and VO2 derivative (e.g. %VO2max)

◦ The calculated modified intensity gets higher when 1) HR level goes closer to HRmax, 2) respiration rate increases 3) when HR increases rapidly

e) Calculating aerobic EPOC using ordinary HR derived intensity

f) Calculating anaerobic EPOC using modified intensity

◦ Modified intensities lower than a predetermined limit (e.g. 80 %VO2max) may be excluded from calculation if only the anaerobic contribution of high intensity work periods is regarded meaningful. (there is always overlap in energy production meaning that even low intensity exercise has little anaerobic contribution. Anaerobic contribution of energy production increases significantly above anaerobic threshold intensities that is commonly around 80% VO2max)

g) Determining total anaerobic Training Effect by scaling aerobic and anaerobic EPOC values and optionally their derivatives. Training effect classification may be based on commonly known coaching science, i.e. anaerobic work quantities in different exercises. In addition, physical fitness level of a person may be taken into account when evaluating the anaerobic load of the performed exercise. In principle, a person with higher fitness level (or activity level) needs to get higher EPOC to achieve similar training effect;

◦ In similar fashion, the performed aerobic EPOC is calculated and scaled during exercise by comparing measured aerobic EPOC to reference values for aerobic work. Person's physical fitness level may be taken into account when classifying the calculated EPOC. Classification may comprise aerobic training effect having values typically between 1 and 5, and having a verbal description between minor and overreaching training effect.

h) Providing aerobic and anaerobic training effect values or their proportions to the user

i) Although there is no actual interval detection method, it is also possible to calculate time periods that exceed predetermined limit values. For example, periods having modified intensity higher than 100% can be regarded as moderate anaerobic intervals. Periods having modified intensity higher than 115% can be regarded as high intensity anaerobic intervals. Periods having modified intensity higher than 140% can be regarded as high speed anaerobic intervals. These intensity limits can be fixed but preferably they change linearly based on user's fitness level.

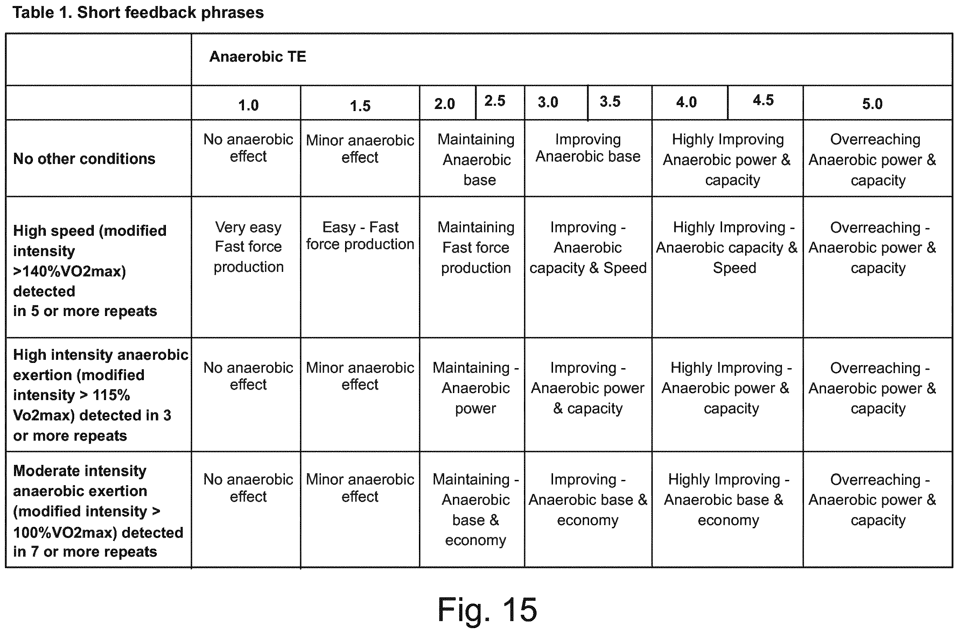

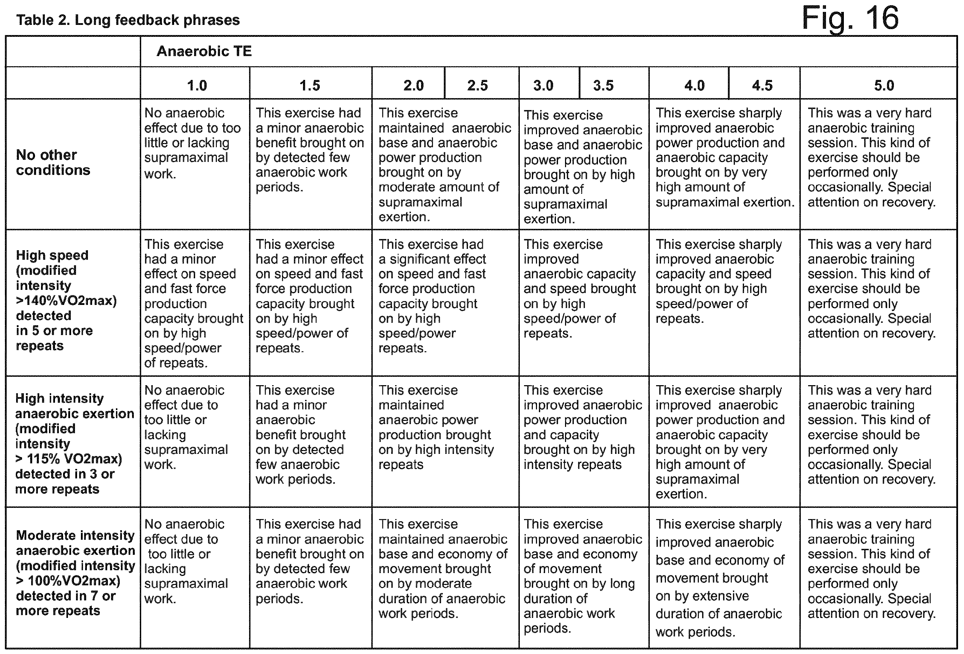

j) When total anaerobic TE and the number of exercise periods (=intervals bouts) above predetermined intensity values are known (at any moment of exercise) it is possible to give feedback sentences (See table 1 in figure 15 and table 2 in figure 16) regarding achieved exercise benefits.

k) Also other characteristics of different exercise periods can be presented to the user, for example average duration and intensity of intervals.

Implementation of calculation without having interval detection as a mandatory step may not have as high requirements for calculation power / memory. Therefore it may be more suitable to be used in commercial wristop computers or heart rate monitors. In addition it may allow better correspondence of results in an end user devices when similar exercises have been done with and without information on external work output - for example on one day user may perform interval workout outside having GPS enabled whereas on another day he/she might perform the workout inside on a treadmill. Of course, user expects that results are similar even if the input parameters used in calculation might be different. In this exemplary embodiment modified intensity based model may be implemented in a way that it combines information on HR and external work output (GPS) to provide final estimate of intensity (modified intensity). Of course, HR based model works solely using HR information. Having HR information included in both models stabilizes results: For example, results from treadmill workout (without speed information) correspond well with outside running results (with speed information). This approach may also stabilize results because both HR and external work output signals may always include error peaks even if various artefact correction algorithms are applied. Averaging may correct error peaks on one part. In addition, model may be implemented in a way that boosting effect for external work output estimate (= modified intensity; can be calculated either solely based on heart rate or solely based on theoretical VO2 or by combining HR information with theoretical VO2 information) is applied only when both measures show similar trends: E.g. detected high speed peaks in GPS signal may be excluded if HR trend does not show the same phenomenon or vice versa.

In one exemplary embodiment combination of aerobic and anaerobic training effects is utilized in determining recovery time from exercise. Tables 3 and 4 show example of how recovery time can be linked to different TE values. In one exemplary embodiment higher one of recovery values is exposed to the user.

Table 3. Example of recovery time accumulation with respect to different aerobic training effect values

| Training effect | Recovery time in hours | Training effect | Recovery time in hours |
|---|---|---|---|
| 1.0: | 0.1 | 3.0: | 19.4 |
| 1.1: | 1.0 | 3.1: | 20.4 |
| 1.2: | 2.0 | 3.2: | 21.3 |
| 1.3: | 3.0 | 3.3: | 22.3 |
| 1.4: | 3.9 | 3.4: | 23.3 |
| 1.5: | 4.9 | 3.5: | 24.2 |
| 1.6: | 5.9 | 3.6: | 26.4 |
| 1.7: | 6.8 | 3.7: | 28.8 |
| 1.8: | 7.8 | 3.8: | 31.2 |
| 1.9: | 8.8 | 3.9: | 33.6 |
| 2.0: | 9.7 | 4.0: | 36.0 |
| 2.1: | 10.7 | 4.1: | 38.4 |
| 2.2: | 11.7 | 4.2: | 40.8 |
| 2.3: | 12.6 | 4.3: | 43.2 |
| 2.4: | 13.6 | 4.4: | 45.6 |
| 2.5: | 14.6 | 4.5: | 48.0 |
| 2.6: | 15.5 | 4.6: | 52.8 |
| 2.7: | 16.5 | 4.7: | 57.6 |
| 2.8: | 17.5 | 4.8: | 62.4 |
| 2.9: | 18.4 | 4.9: | 67.2 |
|  |  | 5.0: | 72.0 |

Table 4. Example of recovery time accumulation matrix with respect to different anaerobic training effect values and detected intensities.

|  | Anaerobic TE | | | | | |
|---|---|---|---|---|---|---|
|  | 1.0-1.4 | 1.5-1.9 | 2.0-2.9 | 3.0-3.9 | 4.0-4.9 | 5.0 |
| No other conditions | Rec time 0-11h | Rec time 12-23h | Rec time 24-47h | Rec time 48-71h | Rec time 72-95h | Rec time 96h |
| High speed or power detected in several repeats | Rec time 0-17h | Rec time 17-35h | Rec time 36-59h | Rec time 60-81h | Rec time 82-95h | Rec time 96h |
| Moderate anaerobic exertion detected in several repeats | Rec time 0-11h | Rec time 12-23h | Rec time 24-47h | Rec time 48-71h | Rec time 72-95h | Rec time 96h |
| Easy anaerobic exertion detected in several repeats | Rec time 0-11h | Rec time 12-23h | Rec time 24-47h | Rec time 48-71h | Rec time 72-95h | Rec time 96h |

In one exemplary embodiment anaerobic recovery time is calculated as a function of anaerobic TE value (see table 5). In addition to that high speed periods may be weighted in a way that they may boost recovery time upwards with additional recovery time

Additional recovery time may accumulate as follows:

Additional recovery time in minutes = 10 * time over 140%VO2max (sec) + 3.33 * time over 115%VO2max (sec)

In one exemplary embodiment maximum additional recovery time is 24h. Accordingly, an exercise with 3.0 aerobic training effect, 3.0 anaerobic training effect and 150 second of exercise above 140%VO2max would produce 25.8h + 24h = 49,8h of recovery time. Table 5. Example of recovery time accumulation with respect to different anaerobic training effect values.

| Anaerobic Training Effect | Anaerobic recovery time in hours | | Anaerobic Training Effect | Anaerobic recovery time in hours |
|---|---|---|---|---|
| 0-0.9 | 0 | | 3 | 25.8 |
| 1 | 0.1 | | 3.1 | 27.1 |
| 1.1 | 1.4 | | 3.2 | 28.4 |
| 1.2 | 2.7 | | 3.3 | 29.7 |
| 1.3 | 3.9 | | 3.4 | 30.9 |
| 1.4 | 5.2 | | 3.5 | 32.2 |
| 1.5 | 6.5 | | 3.6 | 35.1 |
| 1.6 | 7.8 | | 3.7 | 38.3 |
| 1.7 | 9.1 | | 3.8 | 41.5 |
| 1.8 | 10.4 | | 3.9 | 44.7 |
| 1.9 | 11.7 | | 4 | 47.9 |
| 2 | 12.9 | | 4.1 | 51.1 |
| 2.1 | 14.2 | | 4.2 | 54.3 |
| 2.2 | 15.5 | | 4.3 | 57.5 |
| 2.3 | 16.8 | | 4.4 | 60.6 |
| 2.4 | 18.1 | | 4.5 | 63.8 |
| 2.5 | 19.4 | | 4.6 | 70.2 |
| 2.6 | 20.7 | | 4.7 | 76.6 |
| 2.7 | 21.9 | | 4.8 | 83.0 |
| 2.8 | 23.2 | | 4.9 | 89.4 |
| 2.9 | 24.5 | | | |
| 5 | 95.8 | | | |

In one exemplary embodiment the described invention is applied during automatically guided workouts where user exercises with a wristop computer, mobile phone or other similar device. In such a case user may select a target training effect for the workout or the target is selected automatically from e.g. a training plan. During the workout guidance is given to the user by utilizing either auditory (voice guidance), visual (guidance using text, pictures or symbols) or kinesthetic (vibration) feedback. The content of feedback helps the user in reaching the target in a comfortable way. In addition to target training effect, also training duration and/or distance can be preset. Exercise bank can be utilized in the way that several different exercise types are optional to the user: for example steady pace exercises, long intervals, and short intervals.

Figure 3 presents an exercise having a plurality of intensity intervals. Usually a starting edge d1 (rising derivative), amount a, falling edge d2 (falling derivative) and duration 1 of a intensity interval are clearly visible in a HR/time-chart. These are characteristics of that intensity interval. A straightforward manner to determine the anaerobic training effect achieved during the exercise is to determine each interval with its starting and ending points and its intensity as well as duration by using memory buffer during recorded exercise. After detection of these parameters anaerobic training effect can be determined by utilizing information on interval duration as well as different weighting methods described in Figure 4. However, that kind of calculation would need still a lot of hardware resources.

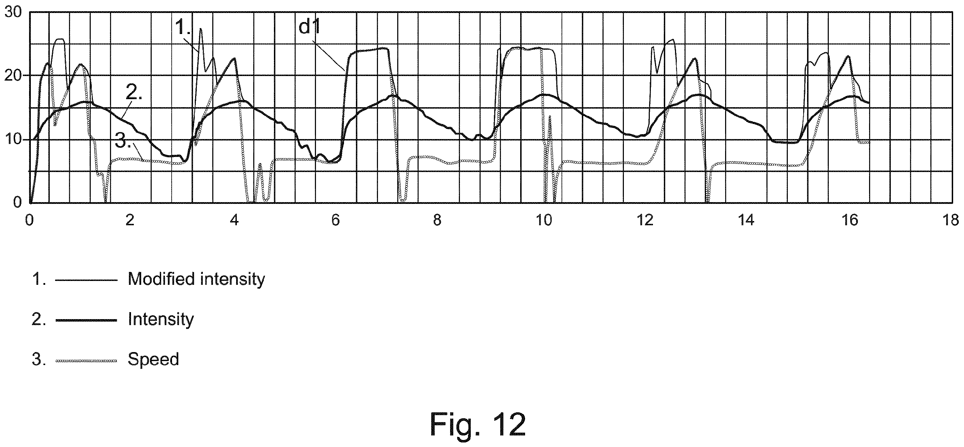

Figure 12 presents results of an intelligent calculation for anaerobic intensity in an exercise using minimum amount of memory. An ordinary training effect TE is calculated as taught in US 7,192,401 B2 which is incorporated herein. Intensity is monitored by a heart rate sensor and preferably by another sensor sensing output power, like speed. The ordinary training effect TE (may be in terms of EPOC) is determined in a known manner. This disclosure presents now a method for determining an anaerobic training effect in same terms.

Referring to figure 12 the exercise has six intervals during 16 minutes, each interval lasting about one minute. Intensity (%VO2max), line 2 has been measured indirectly from heart beat signal. External workload, here speed has been monitored by GPS. The ordinary intensity, line 2 gives quite a near repeating curve. Then measuring speed is much more challenging, when there are breaks in signal. Only the third interval has a clear curve of the speed corresponding to the actual workout. In all other intervals GPS-signal has been broken. However, whenever speed information is available, it may precede intensity data based on heart rate when calculating modified intensity. Thus, in the third interval the curves of speed and modified intensity coincide.

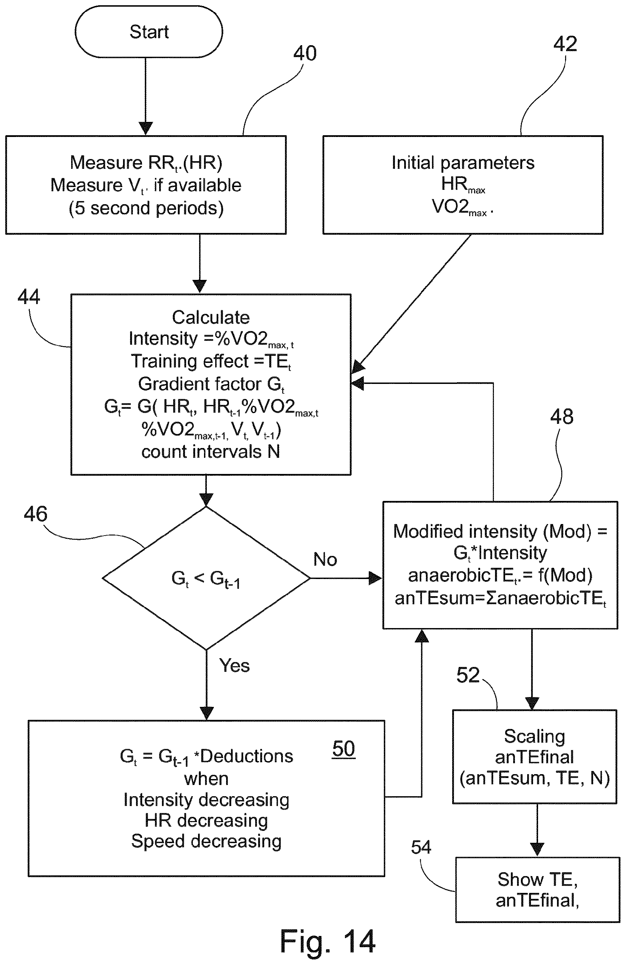

An embodiment according to the invention is shown in Figure 14.

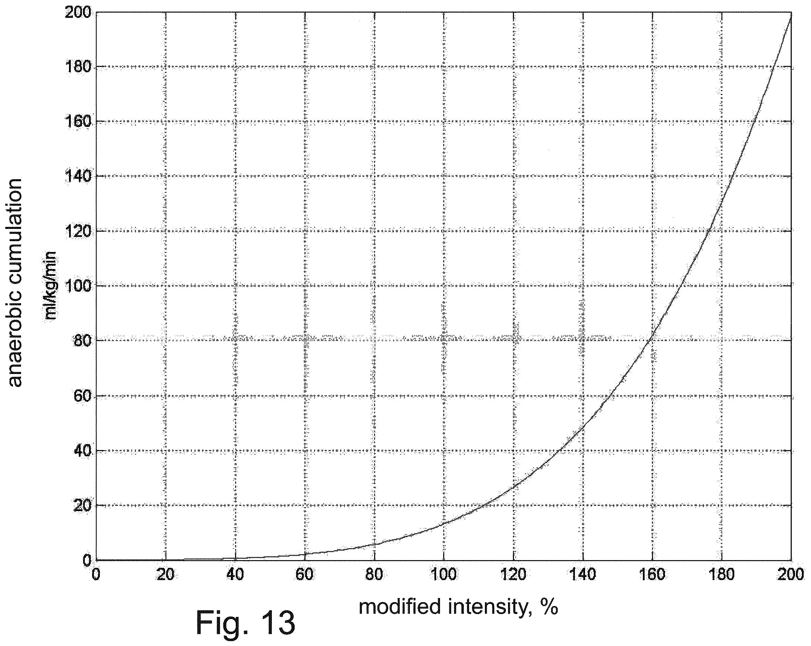

Modified intensity is determined using an ordinary HR derived %VO2max estimate, %HRmax and external workload after every 5 seconds with a range between e.g. 85-200%.Modified intensity is then converted to the accumulation of anaerobic training effect (anTE) using an empiric function shown graphically in Figure 13. It counts the anTE adding each new value of a five second window to a sum, which presents a total anaerobic training effect, but without scaling.. Generally the calculation of modified intensity takes place in short periods of 2 - 20 seconds, while a 3 - 10 second period may be better.

The intelligence of the above described method is based on minimum information about characteristics of each interval and using just a calculation window without full history data of exercise. The characteristics of each interval is revealed just by a derivate of intensity and a starting level, and intensity change calculated preferably in a simply manner as a continuous average value. The full characteristics of each exercise interval are never revealed, but necessary information of each interval is obtained indirectly in a continuous calculation.

Referring to Figure 14 heart rate (RRt) and external workload like speed V is measured periodically, e.g. in 5 second periods (generally 0.5 - 15 second, preferably 1- 6 seconds). There are background information entered initially, particularly values depicting the maximum heart rate and the fitness level of the user,

The modified intensity is determined by a multiplication of a factor $G_t$ and the measured intensity, i.e. the ordinary intensity. The factor $G_t$ is calculated continuously and it has initial value of one as long as a gradient function yields a higher value over 1 (100%) using both the increasing gradient value and a starting level, step 44. The illustration of Figure 14 is schematic. Artefact corrections are not presented. When the signal quality is weakening, the modified intensity is calculated more conservatively i.e. the factor G is reduced.

The level at which intensity ends up at any measurement point can be taken into account for example by using weights for multiplication of the actual gradient. A basic level of 50% yields weight of 0.35, 85% gives a weight of 1, 90% 1.12, and finally 100% level gives a weight of 1.4. A gradient value is easily obtained as a difference of two sequential values (time difference always 5 seconds). The weight is further multiplied by the MET -difference between current and previous measurement point. For example, if intensity ends up to 85% level and has increased by 2METs from previous point then G-value is 1.00 x 2 =2.00.

A new value is calculated for the factor $G_t$ in every period (step 46). If the new value is bigger, an anaerobicTE-speed can been measured imminently, step 48. Otherwise there is a deduction process decrease the value of the factor G until it is one. The deduction are based on decreasing intensity, decreasing heart rate and/or decreasing external workload, step 50

Thus, another aspect is that factor G is kept up until it is gradually reduced due to several different factors like decreasing intensity (step 50), decreasing heart rate and/or decreasing external workload. Preferably speed or other power output is measured, when that information precedes heart rate based intensity. After the deduction phase, step 50, the modified intensity (Mod) is calculated first in step 48. The function in Figure 13 yields the accumulation speed of the anaerobic training effect, f(Mod), particularly its upslope component. Unscaled anaerobic training effect (anaerobic $TE_t$) is obtained by integrating all values to a sum. The recovery (a downslope component) is omitted and it can be handled in a similar manner as related to the aerobic TE (US7192401 B2) herein incorporated), if desired.

The "modified intensity" method according to Figure 14 with recovery calculation (downslope component) has an advantage of simplicity, when the aerobic training effect is calculated using dependency between an intensity/oxygen consumption and EPOC. Now a special calculation of heart rate or measuring of external work load reveals actual and temporal oxygen requirement of exercise (theoretical VO2), which means that temporally physiological intensity (sum of aerobic and anaerobic energy yield) can be far over 100 % VO2MAX - here limited TO 200% in this exemplary embodiment. Using same Figure 13 (or similar chart) both aerobic intensity and anaerobic intensity can be converted to momentary EPOC ("oxygen debt") - values being so called upslope components. When downslope component is same for both, the overall calculation need relatively little resources in addition to the ordinary TE-calculation.

In order to standardize the result being compatible to different sport and different number of intervals, step 52, the result is scaled using also an ordinary training effect and the number of executed intervals. The scaling can be accomplished in many ways.

Finally the result is displayed in step 54, when both ordinary training effect and anaerobic training effect are shown in a display.

In one exemplary embodiment modified intensity is calculated as:

$$\text{modified intensity} = \text{Anaerobic Multiplier (G)}*\text{intensity\_t,}$$

where

$$\text{anaerobic multiplier (Gt)} = 1.3841*\text{intensity\_t}^2*(\text{MET\_t-MET\_t-1}) \text{ and intensity\_t is}$$

$$\text{provided as \%VO2max.}$$

The anaerobic multiplier (Gt) is based, in each period on final maximum intensity in selected power of range 1- 4 (typically 2) and an increase of intensity within the period.

If external workload (Speed & altitude or power output) is recorded it may be heavily weighted in the calculation of modified intensity. In one exemplary embodiment modified intensity may be calculated solely based on external workload if following conditions are fulfilled: Anaerobic multiplier is greater than 1 and external workload is between 100 and 200%VO2max.

In one exemplary embodiment modified intensity may be downgraded in cases when recorded intensity has been continuously high. For example in cases when intensity has not been under 70%VO2max any time in preceding 5min period. This rule may be used as a "sanity check " for the modified intensity especially in cases when external workload information is not available since it is impossible to perform significant amount of anaerobic work if there are no recovery breaks during in short term history .

A practical example of calculation of modified intensity and EPOC when external workload data is not available:

A Runner has VO2max of 52.5 ml/kg min which is equivalent to 15 METs (= his VO2max is 15-fold when compared to his expected resting VO2 of 3.5 ml/kg/min). The runner starts an exercise during which he runs 100m repeats. In this example EPOC/TE accumulation is described in detail regarding the first repeat that lasts 15 seconds:

- During the first 5 seconds ordinary heart rate based intensity increases from 30%VO2max to 50%, which corresponds to an increase in METs from 4,5 to 7,5MET. So the increase is 3METs and correspondingly

   ○

$$\text{anaerobic multiplier (G)} = 1.3841*0.5^2*3 = 1.03$$

   ○

$$\text{modified intensity} = 1.03*50\%\text{VO2max} = 52\%\text{VO2max}$$

   ○ Because modified intensity is lower than 85%, 50% intensity is returned.
   ■ In this exemplary embodiment only intensities above 85%VO2max are regarded meaningful in accumulating anaerobic training effect
   ○ anaerobic EPOC accumulates by 0.1002 ml/kg
   ○ aerobic EPOC accumulates by 0.1002 ml/kg

- During second 5 sec period intensity increase from 50%VO2max to 70%VO2max, which corresponds to an increase in METs from 7.5 to 10.5MET. So the increase is 3METs and correspondingly

○

$$\text{Anaerobic multiplier} = 1.3841*0.7\text{\textasciicircum}2*3 = 2.03$$

○

$$\text{modified intensity} = 2.03*70\%\text{VO2max} = 142\%\text{VO2max}$$

○ anaerobic EPOC accumulates from 0.1002 ml/kg to 4.1352 ml/kg
○ aerobic EPOC accumulates from 0.1002 ml/kg to 0.3967 ml/kg

- During third 5sec period intensity increases from 70%VO2max to 80%VO2max, which corresponds to an increase in METs from 10.5 to 12 METs. So the increase is 1,5 METs and correspondingly

○

$$\text{anaerobic multiplier} = 1.3841*0.8\text{\textasciicircum}2*1.5 = 1.33$$

○

$$\text{modified intensity} = 1.33*80\%\text{VO2max} = 106\%\text{VO2max}$$

○ anaerobic EPOC accumulates from 4.1352 ml/kg to 5.5195 ml/kg
○ aerobic EPOC accumulates from 0.3967 ml/kg to 0.8738 ml/kg

Total accumulated EPOCs are: anaerobic EPOC = 5.5195 ml/kg/min, aerobic EPOC 0.8738 ml/kg/min. Difference is 4.6457 ml/kg/min which would mean accumulated anaerobic training effect value of 1.2 after the first repeat when runner's activity class is 7. When EPOC is used as an anaerobic sum measure scaling logic of figure 5 cannot be used as such. One suitable scaling logic is disclosed in patent publication US7805186 (B2) which presents an exemplary dependency between EPOC, activity class and Training effect.

In one exemplary embodiment, a method and system for detecting exercise intervals according to the present invention may include defining the interval-likeness of a time sequence of a physiological parameter, wherein the interval-likeness is proportional to at least some of the following properties of the time sequence: the time derivatives within the sequence, the local minima and maxima within the sequence, and the fluctuations within the sequence. A time sequence may then be regarded as an exercise interval if its interval-likeness value is higher than a predetermined threshold value.

In further exemplary embodiments, methods and systems for detecting exercise intervals, analyzing anaerobic exercise periods, and analyzing training effects may be described. A physiological response of a user may be continuously measured through one or more physiological parameters, wherein the physiological parameters may be recorded as physiological values. One or more high intensity intervals and non-interval periods may be identified based on a degree of change of one or more of the physiological values over a period of time. An anaerobic sum may be defined from at least one of: high intensity intervals and non-interval periods based on their properties. An anaerobic training effect may be determined based on anaerobic sum and a user's background parameters. The anaerobic training effect may be displayed to the user in comparison with calculated aerobic training effect.

As would be understood by a person of ordinary skill in the art, the training effect may be displayed in any manner as would be understood by a person or ordinary skill in the art. In further exemplary embodiments, the number of identified high intensity intervals, the duration of the intervals, or the like may be displayed to the user. In further exemplary embodiments, high intensity intervals may be classified and presented to a user according to predetermined criteria in any manner as would be understood in the art. In further exemplary embodiments, a description of the exercise and the physiological effect may be provided in any manner as would be understood by a person of ordinary skill in the art.

In further exemplary embodiments, methods and systems for analyzing anaerobic exercise periods, and analyzing training effects may be disclosed. A physiological response of a user may be continuously measured through one or more physiological parameters, wherein the physiological parameters may be recorded as physiological values. An external workload may be continuously measured wherein a plurality of measured workload values may be recorded

and each measured workload value may be associated with one or more of the measured physiological values to form a plurality of data points. An aerobic training load may be calculated based on a measured physiological response. An anaerobic training load may be calculated based on measured external workload. A total training effect is determined using the higher training load value and one or more of user's background parameters, and determining anaerobic training effect as a relative value according to comparison between anaerobic training effect and total training effect.

In further exemplary embodiments, high intensity intervals may be identified based on at least one of: measured physiological response and measured external workload. The high intensity intervals may be classified based on predetermined criteria, and the number of identified high intensity intervals, duration of the intervals, and the classification of high intensity intervals may displayed to the user. In further exemplary embodiments, a description of the exercise and the physiological effect may be provided in any manner as would be understood by a person of ordinary skill in the art.

In still further exemplary embodiments, methods and systems for detecting exercise intervals, analyzing anaerobic exercise periods, and analyzing training effects may be disclosed. A physiological response of a user may be continuously measured through one or more physiological parameters, wherein the physiological parameters may be recorded as physiological values. An external workload may be continuously measured, wherein measured workload values may be recorded and each measured workload value may be associated with one or more of the measured physiological values to form a plurality of data points. One or more high intensity intervals may be identified based on a degree of change of one or more of the physiological and/or external workload values over a period of time. One or more identified high intensity intervals may be determined to be an anaerobic interval based on one or more factors. An anaerobic sum of the one or more anaerobic intervals may be determined, and anaerobic training effect may be determined by comparing the anaerobic sum with an anaerobic work scale.

In still further exemplary embodiments, methods and systems for detecting exercise intervals, analyzing anaerobic exercise periods, and analyzing training effects may be disclosed. A physiological response of a user may be continuously measured through a plurality of physiological parameters, wherein the plurality of physiological parameters may be recorded as physiological values. An external workload may be continuously measured, wherein a plurality of measured workload values may be recorded, and each measured workload value may be associated with one or more of the measured physiological values to form a plurality of data points. One or more data points may be filtered based on predetermined criteria to form a plurality of accepted data points. One or more high intensity intervals may be identified based on a degree of change of one or more of the physiological or external workload values over a period of time. A probability that the one or more identified high intensity intervals is an anaerobic interval may be calculated based on one or more factors. The one or more high intensity intervals may be classified as an anaerobic interval if the calculated probability is above a predetermined threshold. An anaerobic sum of the one or more anaerobic intervals and the anaerobic sum of non-interval periods may be defined. An aerobic sum of the aerobic intervals may also be defined. Anaerobic training effect may be determined by comparing the anaerobic sum with an anaerobic work scale and an aerobic training effect may be determined by comparing aerobic sum with aerobic work scale. A total training effect may be determined as being the higher training effect value, and a ratio between the anaerobic training effect and the aerobic training effect may be determined, the ratio may represent the proportional benefit of exercise on, for example, energy production pathways.

The foregoing description and accompanying figures illustrate the principles, preferred embodiments and modes of operation of the invention. However, the invention should not be construed as being limited to the particular embodiments discussed above. Additional variations of the embodiments discussed above will be appreciated by those skilled in the art.

Therefore, the above-described embodiments should be regarded as illustrative rather than restrictive. Accordingly, it should be appreciated that variations to those embodiments can be made by those skilled in the art without departing from the scope of the invention as defined by the following claims.

## Claims

1. A computer-implemented method for determining an anaerobic training effect of a user in an exercise using a device with a processor (62), memory (61.1, 61.2) and software, where the exercise contains intensity intervals and at least one anaerobic phase, the method comprising:

   determining and storing a value depicting a fitness level of user and a value depicting a maximum heart rate of the user;
   continuously measuring heart rate by a heart sensor (72) and recording selected number of measured values;
   obtaining intensity values depicting exercise intensity alternatively by calculating continuously an exercise intensity using a measured heart rate value and recording selected number of calculated intensity values and/or measuring external workload; the method further comprising:

determining characteristics of each intensity interval;
determining each intensity interval period from the recorded values of exercise intensity values, where an intensity interval period is detected by determined characteristics of an intensity interval having:

at least one derivative (d1, d2) of intensity; and
at least one of:

an amount (a) of intensity increase during the intensity interval period;
a duration (1) of the intensity interval period ; and
an external workload during the intensity interval period;

calculating an anaerobic training effect using the determined characteristics of the determined intensity interval periods using steps of:

determining continuously a modified intensity value using the measured intensity value and the determined characteristics of each intensity interval;
calculating a variable depicting an accumulation speed of the anaerobic training effect using said modified intensity; and
integrating the accumulation speed and obtaining an unscaled anaerobic training effect, which is optionally scaled being compatible to different sport and different number of intervals; and
outputting the calculated anaerobic training effect to an output device (45).

2. The method according to claim 1, further comprising,

determining a multiplication factor to obtain the modified intensity value from the measured intensity value, the multiplication factor being based on at least an increasing derivate of intensity and starting level of an increase; and
deduction components decreasing the multiplication factor, including at least decreasing intensity.

3. The method according to claim 1 or 2, wherein the intensity value and the modified intensity value are calculated using external workload.

4. The method according to any of claims 1 - 3, further comprising:
scaling of the calculated anaerobic training effect by at least the aerobic training effect and number of intervals obtaining a scale compatible for any number of intervals.

5. The method according to any of claims 1 - 4, further comprising:
scaling of the calculated anaerobic training effect by at least one of total duration or number of intervals.

6. The method according to any of claims 1 - 5, wherein the calculation of the modified intensity value takes place in short periods of 0,5 - 15 seconds.

7. The method according to any of claims 1 -7, wherein the multiplication factor is based, in each interval on final peak intensity in selected power of range 1- 4 and an increase of intensity within the period.

8. The method according to any of claims 1 - 7 further wherein accuracy of intensity measuring is increased by measuring external workload

9. The method according to any of claims 1 - 8, wherein the output power is measured by a GPS-system, an accelerometer or by a power meter.

10. An apparatus for determining an anaerobic training effect of a user in an exercise comprising a device with a processor (62), memory (61.1, 61.2) and software, the exercise containing intensity intervals and at least one anaerobic phase,
the apparatus further comprising:

- a user interface (78) determining and storing a value depicting a fitness level of user and a value depicting a maximum heart rate of the user:

- a heart rate sensor (72) for continuously measuring heart rate and means for recording selected number of measured values,
- means (70) for obtaining intensity values depicting exercise intensity alternatively by calculating continuously an exercise intensity using a measured heart rate value and recording selected number of calculated intensity values and/or means for measuring external workload depicting exercise intensity ; wherein the apparatus has
- means (62) for determining characteristics of each intensity interval by determining each intensity interval period from the recorded values of exercise intensity values, intensity interval having:

○ at least one derivative (d1, d2) of intensity interval period; and
○ at least one of:

▪ an amount (a) of intensity increase during each intensity interval period;
▪ a duration (1) of each intensity interval period; and
▪ an external workload depicting momentary exercise intensity during each intensity interval period;

- means for calculating an anaerobic training effect using the determined characteristics of determined intensity interval periods in steps of
calculating an anaerobic training effect using the determined characteristics of the determined intensity interval periods; the determination of anaerobic training effect using steps of:

determining continuously a modified intensity value using the measured intensity value and the determined characteristics of each intensity interval;
calculating a variable depicting an accumulation speed of the anaerobic training effect using said modified intensity; and
integrating the accumulation speed and obtaining an unscaled anaerobic training effect, which is optionally scaled being compatible to different sport and different number of intervals;
and

- an output device (45) to show the calculated anaerobic training effect to the user.

11. An apparatus according to claim 10, **characterized in that** the apparatus is implemented in one of following: heart rate monitor, fitness device, mobile phone, PDA device, wristop computer or personal computer having software for implementing said software means and hardware for execution of the software.

12. An apparatus according to claim 10 or 11, **characterized by** at least one sensor belonging to group a GPS-system, an accelerometer or by a power meter arranged to measure external workload.

13. An apparatus according to any of claims 10 - 12, **characterized by** further means for calculating continuously an multiplication factor Gt to convert intensity to modified intensity obtaining an accumulation variable for the training effect.


**Patentansprüche**

1. Ein computerimplementiertes Verfahren zum Bestimmen eines anaeroben Trainingseffekts eines Benutzers in einer Übung unter Verwendung eines Geräts mit einem Prozessor (62), einem Speicher (61.1, 61.2) und einer Software, wobei die Übung Intensitätsintervalle und mindestens eine anaerobe Phase enthält, das Verfahren umfassend:

Bestimmen und Speichern eines Wertes, der ein Fitnessniveau des Benutzers darstellt, und eines Wertes, der eine maximale Herzfrequenz des Benutzers darstellt;
kontinuierliche Messung der Herzfrequenz durch einen Herzsensor (72) und Aufzeichnung der ausgewählten Anzahl von Messwerten;
Erhalten von Intensitätswerten, die die Übungsintensität darstellen, alternativ durch kontinuierliches Berechnen einer Übungsintensität unter Verwendung eines gemessenen Herzfrequenzwerts und Aufzeichnen einer ausgewählten Anzahl berechneter Intensitätswerte und/oder Messen der externen Arbeitsbelastung;
Verfahren, ferner umfassend:

Bestimmen der Eigenschaften jedes Intensitätsintervalls;

Bestimmen jeder Intensitätsintervallperiode aus den aufgezeichneten Werten von Übungsintensitätswerten, wobei eine Intensitätsintervallperiode durch bestimmte Eigenschaften eines Intensitätsintervalls erfasst wird mit:

mindestens eine Ableitung (d1, d2) der Intensität; und
mindestens eines von:

einem Betrag (a) des Intensitätsanstiegs während des Intensitätsintervallzeitraums;
einer Dauer (1) der Intensitätsintervallperiode; und
einer externe Arbeitsbelastung während des Intensitätsintervallzeitraums;

Berechnen eines anaeroben Trainingseffekts unter Verwendung der bestimmten Eigenschaften der bestimmten Intensitätsintervallperioden unter Verwendung der folgenden Schritte:

kontinuierliches Bestimmen eines modifizierten Intensitätswerts unter Verwendung des gemessenen Intensitätswerts und der bestimmten Eigenschaften jedes Intensitätsintervalls;
Berechnen einer Variablen, die eine Akkumulationsgeschwindigkeit des anaeroben Trainingseffekts unter Verwendung der modifizierten Intensität darstellt; und
Integrieren der Akkumulationsgeschwindigkeit und Erhalten eines nicht skalierten anaeroben Trainingseffekts, der optional skaliert wird,um mit verschiedenen Sportarten und verschiedenen Intervallen kompatibel zu sein;; und
Ausgabe des berechneten anaeroben Trainingseffekts an ein Ausgabegerät (45).

2. Verfahren nach Anspruch 1, ferner umfassend:

Bestimmen eines Multiplikationsfaktors, um den modifizierten Intensitätswert aus dem gemessenen Intensitätswert zu erhalten, wobei der Multiplikationsfaktor auf mindestens einer zunehmenden Ableitung der Intensität und dem Startniveau einer Zunahme basiert; und
Abzug von Komponenten, die den Multiplikationsfaktor verringern, einschließlich mindestens abnehmender Intensität.

3. Verfahren nach Anspruch 1 oder 2, wobei der Intensitätswert und der modifizierte Intensitätswert unter Verwendung einer externen Arbeitsbelastung berechnet werden.

4. Verfahren nach einem der Ansprüche 1 bis 3, ferner umfassend:
Skalieren des berechneten anaeroben Trainingseffekts durch mindestens den aeroben Trainingseffekt und der Anzahl der Intervalle, wobei eine Skala erhalten wird, die für eine beliebige Anzahl von Intervallen kompatibel ist.

5. Verfahren nach einem der Ansprüche 1 bis 4, ferner umfassend:
Skalieren des berechneten anaeroben Trainingseffekts um mindestens eine der Gesamtdauer oder Anzahl der Intervalle.

6. Verfahren nach einem der Ansprüche 1 bis 5, wobei die Berechnung des modifizierten Intensitätswerts in kurzen Zeiträumen von 0,5 bis 15 Sekunden erfolgt.

7. Verfahren nach einem der Ansprüche 1 bis 7, wobei der Multiplikationsfaktor in jedem Intervall auf der endgültigen Spitzenintensität in der ausgewählten Leistung des Bereichs 1 bis 4 und einer Intensitätserhöhung innerhalb des Zeitraums basiert.

8. Verfahren nach einem der Ansprüche 1 bis 7, wobei die Genauigkeit der Intensitätsmessung durch Messen der externen Arbeitsbelastung erhöht wird.

9. Verfahren nach einem der Ansprüche 1 bis 8, wobei die Ausgangsleistung durch ein GPS-System, einen Beschleunigungsmesser oder durch einen Leistungsmesser gemessen wird.

10. Vorrichtung zum Bestimmen eines anaeroben Trainingseffekts eines Benutzers in einer Übung, umfassend eine Vorrichtung mit einem Prozessor (62), einem Speicher (61,1, 61,2) und einer Software, wobei die Übung Intensitätsintervalle und mindestens eine anaerobe Phase enthält,

wobei die Vorrichtung ferner Folgendes umfasst:

- eine Benutzerschnittstelle (78), die einen Wert bestimmt und speichert, der ein Fitnessniveau des Benutzers darstellt, und einen Wert, der eine maximale Herzfrequenz des Benutzers darstellt:
- einen Herzfrequenzsensor (72) zum kontinuierlichen Messen der Herzfrequenz und Mittel zum Aufzeichnen einer ausgewählten Anzahl von Messwerten;
- Mittel (70) zum Erhalten von Intensitätswerten, die die Übungsintensität darstellen, alternativ durch kontinuierliches Berechnen einer Übungsintensität unter Verwendung eines gemessenen Herzfrequenzwerts und Aufzeichnen einer ausgewählten Anzahl berechneter Intensitätswerte und/oder Mittel zum Messen der externen Arbeitsbelastung, die die Übungsintensität darstellt;

wobei die Vorrichtung Folgendes umfasst:

- Mittel (62) zum Bestimmen von Eigenschaften jedes Intensitätsintervalls durch Bestimmen jeder Intensitätsintervallperiode aus den aufgezeichneten Werten von Übungsintensitätswerten, wobei das Intensitätsintervall Folgendes aufweist:

  ∘ mindestens eine Ableitung (d1, d2) der Intensitätsintervallperiode; und
  ∘ mindestens eines von:

    ▪ einem Betrag (a) des Intensitätsanstiegs während jedes Intensitätsintervallzeitraums;
    ▪ einer Dauer (1) jeder Intensitätsintervallperiode; und
    ▪ einer externen Arbeitsbelastung, die die momentane Trainingsintensität während jeder Intensitätsintervallperiode darstellt;

- Mittel zur Berechnung eines anaeroben Trainingseffekts unter Verwendung der bestimmten Eigenschaften bestimmter Intensitätsintervallperioden in Schritten von:
Berechnen eines anaeroben Trainingseffekts unter Verwendung der bestimmten Eigenschaften der bestimmten Intensitätsintervallperioden; die Bestimmung des anaeroben Trainingseffekts anhand der folgenden Schritte:

  kontinuierliches Bestimmen eines modifizierten Intensitätswerts unter Verwendung des gemessenen Intensitätswerts und der bestimmten Eigenschaften jedes Intensitätsintervalls;
  Berechnen einer Variablen, die eine Akkumulationsgeschwindigkeit des anaeroben Trainingseffekts unter Verwendung der modifizierten Intensität darstellt; und
  Integrieren der Akkumulationsgeschwindigkeit und Erhalten eines nicht skalierten anaeroben Trainingseffekts, der optional skaliert wird, um mit verschiedenen Sportarten und verschiedenen Intervallen kompatibel zu sein;
  und

- einem Ausgabegerät (45), um dem Benutzer den berechneten anaeroben Trainingseffekt zu zeigen.

11. Vorrichtung nach Anspruch 10, **dadurch gekennzeichnet, dass** die Vorrichtung in einer der folgenden Situationen implementiert ist: Herzfrequenzmesser, Fitnessgerät, Mobiltelefon, PDA-Gerät, Armbandcomputer oder Personalcomputer mit Software zum Implementieren der Softwaremittel und Hardware zur Ausführung der Software.

12. Vorrichtung nach Anspruch 10 oder 11, **gekennzeichnet durch** mindestens einen Sensor, der zur Gruppe eines GPS-Systems, eines Beschleunigungsmessers oder eines Leistungsmessers gehört, der zur Messung der externen Arbeitslast angeordnet ist.

13. Vorrichtung nach einem der Ansprüche 10 bis 12, **gekennzeichnet durch** weitere Mittel zum kontinuierlichen Berechnen eines Multiplikationsfaktors Gt, um die Intensität in eine modifizierte Intensität umzuwandeln, wobei eine Akkumulationsvariable für den Trainingseffekt erhalten wird.

## Revendications

1. Méthode implantée sur ordinateur pour déterminer un effet d'entraînement anaérobie d'un utilisateur dans un exercice utilisant un dispositif avec un processeur (62), une mémoire (61.1, 61.2) et un logiciel, où l'exercice consiste en des intervalles d'intensité et au moins une phase anaérobie, la méthode consistant à :

déterminer et stocker une valeur représentant le niveau de forme physique de l'utilisateur et une valeur représentant la fréquence cardiaque maximale de l'utilisateur ;
mesurer la fréquence cardiaque de façon continue à l'aide d'un capteur cardiaque (72) et enregistrer un certain nombre de valeurs mesurées ;
obtenir des valeurs d'intensité représentant l'intensité de l'exercice en alternance en calculant en continu une intensité d'exercice à l'aide d'une valeur de fréquence cardiaque mesurée et en enregistrant un nombre sélectionné de valeurs d'intensité calculées et/ou en mesurant la charge de travail externe ;
la méthode consistant en outre à :

déterminer les caractéristiques de chaque intervalle d'intensité ;
déterminer chaque période d'intervalle d'intensité à partir des valeurs enregistrées de valeurs d'intensité de l'exercice, où une période d'intervalle d'intensité est détectée par des caractéristiques déterminées d'un intervalle d'intensité comprenant :

au moins un dérivé (d1, d2) de l'intensité ; et
au moins l'une des caractéristiques suivantes :

une quantité (a) d'augmentation de l'intensité pendant la période d'intervalle d'intensité ;
une durée (1) de la période d'intervalle d'intensité ; et
une charge de travail externe pendant la période d'intervalle d'intensité ;

calculer un effet d'entraînement anaérobie en utilisant les caractéristiques déterminées des périodes d'intervalle d'intensité déterminées en utilisant les étapes consistant à :

déterminer de façon continue une valeur d'intensité modifiée en utilisant la valeur d'intensité mesurée et les caractéristiques déterminées de chaque intervalle d'intensité ;
calculer une variable représentant une vitesse d'accumulation de l'effet d'entraînement anaérobie en utilisant ladite intensité modifiée ; et
intégrer la vitesse d'accumulation et obtenir un effet d'entraînement anaérobie non échelonné, qui est éventuellement échelonné en étant compatible avec différents sports et différents nombres d'intervalles ; et
produire un effet d'entraînement anaérobie calculé vers un dispositif de sortie (45).

2. Méthode conformément à la revendication 1 consistant, en outre, à

déterminer un facteur de multiplication pour obtenir la valeur d'intensité modifiée à partir de la valeur d'intensité mesurée, le facteur de multiplication étant basé sur au moins une dérivée croissante de l'intensité et le niveau de départ d'une augmentation ; et
des éléments de déduction diminuant le facteur de multiplication, comprenant au moins l'intensité décroissante.

3. Méthode conformément à la revendication 1 ou 2, dans laquelle la valeur d'intensité et la valeur modifiée d'intensité sont calculées en utilisant une charge de travail externe.

4. Méthode conformément à l'une des revendications 1 - 3, consistant en outre à :
mettre à l'échelle l'effet d'entraînement anaérobie calculé par au moins l'effet d'entraînement aérobie et le nombre d'intervalles en obtenant une échelle compatible pour un nombre quelconque d'intervalles.

5. Méthode conformément à l'une des revendications 1 à 4, consistant en outre à :
mettre à l'échelle l'effet d'entraînement anaérobie calculé par au moins l'une des durées totales ou le nombre d'intervalles.

6. Méthode conformément à l'une des revendications 1 à 5, dans laquelle le calcul de la valeur modifiée d'intensité a lieu dans de courtes périodes allant de 0,5 à 15 secondes.

7. Méthode conformément à l'une des revendications 1 à 7, dans laquelle le facteur de multiplication est basé, dans chaque intervalle, sur l'intensité de pointe finale dans une puissance de plage 1 à 4 sélectionnée et une augmentation de l'intensité au cours de la période.

8. Méthode conformément à l'une des revendications 1 à 7, selon laquelle la précision de la mesure de l'intensité est augmentée par la mesure de la charge de travail externe.

9. Méthode conformément à l'une des revendications 1 à 8, dans laquelle la puissance de sortie est mesurée par un système GPS, un accéléromètre ou un wattmètre.

10. Appareil pour déterminer un effet d'entraînement anaérobie d'un utilisateur dans un exercice comprenant un dispositif avec un processeur (62), une mémoire (61.1, 61.2) et un logiciel, l'exercice contenant des intervalles d'intensité et au moins une phase anaérobie, l'appareil comprenant en outre :

- une interface utilisateur (78) déterminant et stockant une valeur représentant un niveau de forme physique de l'utilisateur et une valeur représentant une fréquence cardiaque maximale de l'utilisateur :
- un capteur de fréquence cardiaque (72) pour mesurer en continu la fréquence cardiaque et des moyens pour enregistrer un nombre sélectionné de valeurs mesurées,
- des moyens (70) permettant d'obtenir des valeurs d'intensité représentant l'intensité de l'exercice, alternativement en calculant en continu une intensité d'exercice à l'aide d'une valeur de fréquence cardiaque mesurée et en enregistrant un nombre sélectionné de valeurs d'intensité calculées et/ou des moyens permettant de mesurer la charge de travail externe représentant l'intensité de l'exercice ;

dans lequel l'appareil comporte

- des moyens (62) pour déterminer les caractéristiques de chaque intervalle d'intensité en déterminant chaque période d'intervalle d'intensité à partir des valeurs enregistrées des valeurs d'intensité de l'exercice, intervalle d'intensité comportant :

  ◦ au moins un dérivé (d1, d2) de période d'intervalle d'intensité ; et
  ◦ au moins l'une des caractéristiques suivantes :

    ▪ une quantité (a) d'augmentation de l'intensité pendant la période d'intervalle d'intensité ;
    ▪ une durée (1) de chaque période d'intervalle d'intensité ; et
    ▪ une charge de travail externe représentant l'intensité momentanée de l'exercice pendant chaque période d'intervalle d'intensité ;

- des moyens pour calculer un effet d'entraînement anaérobie en utilisant les caractéristiques déterminées de périodes d'intervalle d'intensité déterminées par étapes consistant à
calculer un effet d'entraînement anaérobie en utilisant les caractéristiques déterminées des périodes d'intervalle d'intensité déterminées, déterminer l'effet d'entraînement anaérobie à l'aide d'étapes consistant à :

  déterminer de façon continue une valeur d'intensité modifiée en utilisant la valeur d'intensité mesurée et les caractéristiques déterminées de chaque intervalle d'intensité ;
  calculer une variable représentant une vitesse d'accumulation de l'effet d'entraînement anaérobie en utilisant ladite intensité modifiée ; et
  intégrer la vitesse d'accumulation et obtenir un effet d'entraînement anaérobie non échelonné, qui est éventuellement échelonné en étant compatible avec différents sports et différents nombres d'intervalles ; et

- un dispositif de sortie (45) pour montrer à l'utilisateur l'effet d'entraînement anaérobie calculé.

11. Appareil conformément à la revendication 10, **caractérisé en ce que** l'appareil est mis en œuvre dans l'un des dispositifs suivants : cardiofréquencemètre, dispositif de conditionnement physique, téléphone portable, assistant numérique personnel, montre connectée ou PC doté d'un logiciel permettant l'installation dudit logiciel et du matériel permettant l'exécution du logiciel.

12. Appareil conformément la revendication 10 ou 11, **caractérisé par** au moins un capteur appartenant au groupe d'un système GPS, d'un accéléromètre ou d'un wattmètre agencé pour mesurer une charge de travail externe.

**13.** Appareil conformément à l'une des revendications 10 - 12, **caractérisé par** des moyens supplémentaires pour calculer en continu un facteur de multiplication Gt afin de convertir l'intensité en intensité modifiée obtenant une variable d'accumulation pour l'effet d'entraînement.

Fig. 1

EPOC (ml/kg)
VO2 (ml/kg/min)

80

70 (100%VO2max)

53

35 (50%VO2max)

Speed (km /h)

23

20

15

10

Theoretical VO2 based on speed

HR based VO2

EPOC derived from theoretical VO2 (modified intensity)

EPOC derived from HR based VO2 (ordinary intensity)

Oxygen deficit

Time

Fig. 2

Fig. 3

Fig. 4a

Fig. 4b

Fig. 4c

Fig. 4d

Fig. 5

TRAINING EFFECT DISTRIBUTION

Fig. 6

Fig. 7

Fig. 8

Fig. 9

**Background parameters**

HRmax

36

EPOC_t -1
TRIMP_t -1

Vo2max
and/or activity
level

**Calculation process**

30 — Start

32 — Measure RRI

34 — Artefact correction

HR

RespR

On/off - kinetics information

%HRmax

HR based %VO2max

Δt

HR based EPOC_t TRIMP_t

38

Measure external workload

Theoretical VO2

Modified intensity

Modified intensity based EPOC_t TRIMP_t

Categorization of anaerobic intervals

38

**Outputs**

Output interval count over 140%VO2max and time > 140%VO2max

Output interval count at 115 - 140%VO2max and time > 115%VO2max

Output interval count at 100 - 115%VO2max and time at > 100 - 115%VO2max

40

Output and display verbal description of anaerobic training effect considering physiological effects: anaerobic capacity, anaerobic power, alactic capacity, anaerobic economy, lactate removal capacity, fatigue resistance, neuromuscular performance, speed, fast force development

Anaerobic training effect

Proportion of training effects

Aerobic Training Effect

EP 3 175 782 B1

36

Fig. 10

Fig.11

1. ——————— Modified intensity

2. ——————— Intensity

3. ·············· Speed

Fig. 12

EP 3 175 782 B1

Fig. 13

**Start** 40

Measure $RR_t.(HR)$
Measure $V_t.$ if available
(5 second periods)

42

Initial parameters
$HR_{max}$
$VO2_{max}.$

44

Calculate
Intensity $=\%VO2_{max,t}$
Training effect $=TE_t$
Gradient factor $G_t$
$G_t= G( HR_t, HR_{t-1}\%VO2_{max,t}$
$\%VO2_{max,t-1,} V_{t,} V_{t-1})$
count intervals N

46

$G_t < G_{t-1}$

No

48

Modified intensity (Mod) =
$G_t$*Intensity
anaerobic$TE_t.= f(Mod)$
anTEsum$=\Sigma$anaerobic$TE_t$

Yes

$G_t = G_{t-1}$*Deductions
when
Intensity decreasing
HR decreasing
Speed decreasing

50

52

Scaling
anTEfinal
(anTEsum, TE, N)

54

Show TE,
anTEfinal,

Fig. 14

**Table 1. Short feedback phrases**

| | Anaerobic TE | | | | | | | | |
|---|---|---|---|---|---|---|---|---|---|
| | **1.0** | **1.5** | **2.0** | **2.5** | **3.0** | **3.5** | **4.0** | **4.5** | **5.0** |
| **No other conditions** | No anaerobic effect | Minor anaerobic effect | Maintaining Anaerobic base | | Improving Anaerobic base | | Highly Improving Anaerobic power & capacity | | Overreaching Anaerobic power & capacity |
| **High speed (modified intensity >140%VO2max) detected in 5 or more repeats** | Very easy Fast force production | Easy - Fast force production | Maintaining Fast force production | | Improving - Anaerobic capacity & Speed | | Highly Improving - Anaerobic capacity & Speed | | Overreaching - Anaerobic power & capacity |
| **High intensity anaerobic exertion (modified intensity > 115% Vo2max) detected in 3 or more repeats** | No anaerobic effect | Minor anaerobic effect | Maintaining - Anaerobic power | | Improving - Anaerobic power & capacity | | Highly Improving - Anaerobic power & capacity | | Overreaching - Anaerobic power & capacity |
| **Moderate intensity anaerobic exertion (modified intensity > 100%VO2max) detected in 7 or more repeats** | No anaerobic effect | Minor anaerobic effect | Maintaining - Anaerobic base & economy | | Improving - Anaerobic base & economy | | Highly Improving - Anaerobic base & economy | | Overreaching - Anaerobic power & capacity |

Fig. 15

Table 2. Long feedback phrases

# Fig. 16

EP 3 175 782 B1

| | Anaerobic TE | | | | | | | | |
|---|---|---|---|---|---|---|---|---|---|
| | **1.0** | **1.5** | **2.0** | **2.5** | **3.0** | **3.5** | **4.0** | **4.5** | **5.0** |
| **No other conditions** | No anaerobic effect due to too little or lacking supramaximal work. | This exercise had a minor anaerobic benefit brought on by detected few anaerobic work periods. | This exercise maintained anaerobic base and anaerobic power production brought on by moderate amount of supramaximal exertion. | | This exercise improved anaerobic base and anaerobic power production brought on by high amount of supramaximal exertion. | | This exercise sharply improved anaerobic power production and anaerobic capacity brought on by very high amount of supramaximal exertion. | | This was a very hard anaerobic training session. This kind of exercise should be performed only occasionally. Special attention on recovery. |
| **High speed (modified intensity >140%VO2max) detected in 5 or more repeats** | This exercise had a minor effect on speed and fast force production capacity brought on by high speed/power of repeats. | This exercise had a minor effect on speed and fast force production capacity brought on by high speed/power of repeats. | This exercise had a significant effect on speed and fast force production capacity brought on by high speed/power repeats. | | This exercise improved anaerobic capacity and speed brought on by high speed/power of repeats. | | This exercise sharply improved anaerobic capacity and speed brought on by high speed/power of repeats. | | This was a very hard anaerobic training session. This kind of exercise should be performed only occasionally. Special attention on recovery. |
| **High intensity anaerobic exertion (modified intensity > 115% VO2max) detected in 3 or more repeats** | No anaerobic effect due to too little or lacking supramaximal work. | This exercise had a minor anaerobic benefit brought on by detected few anaerobic work periods. | This exercise maintained anaerobic power production brought on by high intensity repeats | | This exercise improved anaerobic power production and capacity brought on by high intensity repeats | | This exercise sharply improved anaerobic power production and anaerobic capacity brought on by very high amount of supramaximal exertion. | | This was a very hard anaerobic training session. This kind of exercise should be performed only occasionally. Special attention on recovery. |
| **Moderate intensity anaerobic exertion (modified intensity > 100%VO2max) detected in 7 or more repeats** | No anaerobic effect due to too little or lacking supramaximal work. | This exercise had a minor anaerobic benefit brought on by detected few anaerobic work periods. | This exercise maintained anaerobic base and economy of movement brought on by moderate duration of anaerobic work periods. | | This exercise improved anaerobic base and economy of movement brought on by long duration of anaerobic work periods. | | This exercise sharply improved anaerobic base and economy of movement brought on by extensive duration of anaerobic work periods. | | This was a very hard anaerobic training session. This kind of exercise should be performed only occasionally. Special attention on recovery. |

**EP 3 175 782 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014074407 A1
- US 2015250417 A1
- US 2014141937 A1
- US 2015327804 A1
- US 2006032315 A1
- US 7192401 B
- US 7192401 B2
- US 7805186 B2